(19) Europäisches Patentamt
European Patent Office
Office européen des brevets

(11) EP 4 206 255 A1

(12) **EUROPEAN PATENT APPLICATION**
published in accordance with Art. 153(4) EPC

(43) Date of publication:
**05.07.2023 Bulletin 2023/27**

(21) Application number: **21861729.8**

(22) Date of filing: **27.08.2021**

(51) International Patent Classification (IPC):
***C08G 61/02*** *(2006.01)* ***C08G 61/00*** *(2006.01)*
***C08L 33/04*** *(2006.01)* ***C08L 45/00*** *(2006.01)*
***C08L 53/00*** *(2006.01)* ***C08L 53/02*** *(2006.01)*
***C08L 57/02*** *(2006.01)* ***C08L 65/00*** *(2006.01)*
***C08L 93/04*** *(2006.01)* ***C08L 101/00*** *(2006.01)*
***C09J 7/38*** *(2018.01)* ***C09J 11/08*** *(2006.01)*
***C09J 133/00*** *(2006.01)* ***C09J 153/02*** *(2006.01)*
***C09J 201/00*** *(2006.01)*

(52) Cooperative Patent Classification (CPC):
**C07C 39/15; C08G 61/00; C08G 61/02; C08L 33/04; C08L 45/00; C08L 53/00; C08L 53/02; C08L 57/02; C08L 65/00; C08L 93/04; C08L 101/00; C09J 7/38; C09J 11/08; C09J 133/00; C09J 153/02;** (Cont.)

(86) International application number:
**PCT/JP2021/031585**

(87) International publication number:
**WO 2022/045308 (03.03.2022 Gazette 2022/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.08.2020 JP 2020144683**
**28.08.2020 JP 2020144684**
**28.08.2020 JP 2020144685**

(71) Applicant: **SEKISUI CHEMICAL CO., LTD.**
**530-0047 (JP)**

(72) Inventors:
- **OGATA, Yudai**
**Mishima-gun, Osaka 618-0021 (JP)**
- **SHIMOJITOSHO, Akira**
**Mishima-gun, Osaka 618-0021 (JP)**
- **SUGAMI, Yuitsu**
**Mishima-gun, Osaka 618-0021 (JP)**

(74) Representative: **Cabinet Beau de Loménie**
**158, rue de l'Université**
**75340 Paris Cedex 07 (FR)**

(54) **COMPOUND, METHOD FOR PRODUCING COMPOUND, ADHESIVE COMPOSITION AND ADHESIVE TAPE**

(57) The present invention aims to provide a compound capable of increasing the adhesion strength of adhesive compositions, particularly, even to low polarity adherends. The present invention also aims to provide a method for producing the compound, an adhesive composition containing the compound, and an adhesive tape including an adhesive layer containing the adhesive composition. Provided is a compound including: a structural unit (A) derived from a monomer (a) having a solvation free energy $\Delta\mu$ with polytetrafluoroethylene of -30 kcal/mol or less; and a structural unit (B) derived from at least one monomer (b) selected from the group consisting of a terpene monomer, a vinyl monomer, and a conjugated diene monomer.

EP 4 206 255 A1

(52) Cooperative Patent Classification (CPC): (Cont.)
**C09J 201/00**

## Description

TECHNICAL FIELD

The present invention relates to a compound usable for adhesive compositions. The present invention also relates to a method for producing the compound, an adhesive composition containing the compound, and an adhesive tape including an adhesive layer containing the adhesive composition.

BACKGROUND ART

Adhesive tapes have been widely used to fix components in electronic devices. Specifically, for example, adhesive tapes are used to bond a cover panel for protecting a surface of a portable electronic device to a touch panel module or display panel module, or to bond a touch panel module to a display panel module. These adhesive tapes for fixing electronic device components are required to have high adhesion, as well as functions such as heat resistance, heat conductivity, and shock resistance according to the environment in which the tape is used (e.g., Patent Literatures 1 to 3).

CITATION LIST

- Patent Literature

Patent Literature 1: JP 2015-052050 A
Patent Literature 2: JP 2015-021067 A
Patent Literature 3: JP 2015-120876 A

SUMMARY OF INVENTION

- Technical problem

To improve adhesion, known adhesive compositions contain base polymers and tackifier resins. Tackifier resins typically improve adhesion strength by changing the mechanical properties, the surface polarity, and the like of the bulk of the base polymer.

As the application of adhesive tapes has expanded in recent years, adhesive compositions are required to have higher performance. For example, adhesive tapes for fixing electronic device components are becoming thinner and required to have high adhesion strength while being thinner. Meanwhile, adherends have become diversified. Harder-to-bond adherends, such as adherends having relatively low polarity, for example, polyolefin resin adherends, have been used. Conventional adhesive compositions may have insufficient adhesion strength to these adherends.

The present invention aims to provide a compound capable of increasing the adhesion strength of adhesive compositions, particularly, even to low polarity adherends. The present invention also aims to provide a method for producing the compound, an adhesive composition containing the compound, and an adhesive tape including an adhesive layer containing the adhesive composition.

- Solution to problem

The present invention relates to a compound including: a structural unit (A) derived from a monomer (a) having a solvation free energy $\Delta\mu$ with polytetrafluoroethylene of -30 kcal/mol or less; and a structural unit (B) derived from at least one monomer (b) selected from the group consisting of a terpene monomer, a vinyl monomer, and a conjugated diene monomer. The present invention is described in detail below.

The present inventors have successfully produced a novel compound including a structural unit (A) derived from a monomer (a) having a solvation free energy $\Delta\mu$ with polytetrafluoroethylene of a specific value or less and a structural unit (B) derived from at least one monomer (b) selected from the group consisting of a terpene monomer, a vinyl monomer, and a conjugated diene monomer. The present inventors have found out that adding such a compound to an adhesive composition as a tackifier resin can increase the adhesion strength of the adhesive composition, particularly, even to low polarity adherends. The inventors thus completed the present invention.

The compound of the present invention includes a structural unit (A) derived from a monomer (a) having a solvation free energy $\Delta\mu$ with polytetrafluoroethylene of - 30 kcal/mol or less. Hereinafter polytetrafluoroethylene may

also be referred to as "PTFE".

The presence of such a structural unit (A) allows the compound of the present invention to increase the adhesion strength of adhesive compositions. The compound can particularly significantly improve interactions of adhesive compositions with low polarity adherends, thereby increasing adhesion strength even to low polarity adherends. Thus, the compound of the present invention is suitable as a tackifier resin to add to adhesive compositions.

The solvation free energy $\Delta\mu$ with polytetrafluoroethylene herein refers to the standard free energy change when the monomer (a) is brought into contact with polytetrafluoroethylene. A smaller solvation free energy $\Delta\mu$ means stronger interactions between polytetrafluoroethylene and the monomer (a). Polytetrafluoroethylene is known as one of the adherends having the lowest polarity. Strong interactions with polytetrafluoroethylene suggest that the monomer (a) can also significantly improve interactions with low polarity adherends in general. Thus, the presence of the structural unit (A) derived from a monomer (a) having a solvation free energy $\Delta\mu$ with polytetrafluoroethylene of a specific value or less is considered to enable the compound of the present invention to increase the adhesion strength to a wide range of low polarity adherends.

The solvation free energy $\Delta\mu$ of the monomer (a) with polytetrafluoroethylene is not limited as long as it is -30 kcal/mol or less. The solvation free energy $\Delta\mu$ is preferably -40 kcal/mol or less, more preferably -50 kcal/mol or less, still more preferably -60 kcal/mol or less. The lower limit of the solvation free energy $\Delta\mu$ of the monomer (a) with polytetrafluoroethylene is not limited, but is practically -300 kcal/mol. The lower limit is preferably -200 kcal/mol, more preferably -100 kcal/mol.

The solvation free energy $\Delta\mu$ of the monomer (a) with polytetrafluoroethylene can be calculated using molecular dynamics. Specific calculation processes include: (1) molecular modeling; (2) setting charges; (3) creating calculation systems; (4) molecular dynamics calculations; and (5) calculating solvation free energy $\Delta\mu$ by the energy representation method.

(1) Molecular modeling

The chemical structures of PTFE and the monomer (a) are drawn using molecular modeling software (Winmostar (ver. 10) produced by X-Ability Co., Ltd., or its equivalent). At this time, the PTFE as a substrate is regarded as a crystal, and its atomic positions are determined in accordance with Document 1 below.

Document 1: A crystal structure of ultra-dispersed form of polytetrafluoroethylene based on X-ray powder diffraction data, Powder Diffr., Vol. 19, No. 3, September 2004.

(2) Setting charges

The charges for the drawn molecular models are calculated as follows.

For the monomer (a), RESP charges are calculated by density functional calculations (conditions: functional B3LYP/basis function 6-31G**) using quantum chemical calculation software (GAMESS (ver. 2018 R1) produced by the Gordon research group at Iowa State University, or its equivalent).

For the PTFE as a substrate, Lowdin charges are calculated by SCF calculations using first principles calculation software (Quantum ESPRESSO produced by Quantum ESPRESSO Foundation, or its equivalent). SCF calculations are performed under the following conditions. &system

| | |
|---|---|
| ibrav | = 0, |
| nat | = 45, |
| nspin | = 1, |
| ntyp | = 2, |
| ecutwfc | = 25., |
| ecutrho | = 225., |
| occupations | = 'fixed', |
| nosym | = False., |
| noinv | = .False., |
| tot_charge &electrons | = 0., |
| conv_thr | = 1d-6, |
| mixing_beta | = 0.3, |
| mixing_mode | = 'plain', |
| electron_maxstep | = 100, |
| diagonalization | = 'david', |

ATOMIC_SPECIES

C 12.011 C.pbe-rrkjus.UPF
F 18.9984 F.pbe-n-van.UPF

(3) Creating calculation systems

For the PTFE molecular model created above, heptamers are set as basic cells. A PTFE crystal system having a total of 90 PTFE heptamers is created by arranging 10, 3, and 3 basic cells in the x-axis, y-axis, and z-axis directions, respectively, and removing extra atoms. The Z-axis direction corresponds to the polymer extension direction.

A vacuum layer is inserted to the PTFE crystal system. The model of the monomer (a) is placed on a topmost layer of the PTFE in the X-Z plane. At this time, the model of the monomer (a) is placed such that the closest distance from the atoms in the outermost layer of the PTFE is 3 Å. The system used in the later-described calculations for the monomer (a) alone is created by placing one monomer (a) in a cubic cell (999 nm on one side) made of a vacuum layer.

(4) Molecular dynamics calculations

Molecular dynamics calculations (MD calculations) are performed using calculation software (GROMACS (ver. 5.0.7) produced by the University of Groningen, or its equivalent). Energy minimization, MD equilibration, and a production run are performed using the Dreiding force field to calculate the coordinates, speed, and energy of each molecular model.

MD calculations are performed for each of three systems in total: the interface system between the monomer (a) and the PTFE created in (3) Creating calculation systems; the monomer (a) alone; and the PTFE crystal system alone. The calculation conditions are as follows.

[1] System of the PTFE crystal system alone and the interface system between the monomer (a) and the PTFE

Step 1 Energy minimization

Conditions: Integrator = steep, Emtol = 100.0 KJ/mol/nm

Step 2 MD Equilibration

Conditions: NVT ensemble (Nose-Hoover method),
temperature: 300 K, computation time: 2,000 (ps),
time step dt: 1 fs

Step 3 Production run

Conditions: NVT ensemble (Nose-Hoover method),
temperature: 300 K, computation time: 500 (ps)
time step dt: 1 fs

System of the monomer (a) alone

Step 1 Energy minimization

[2] Conditions: Integrator = steep, Emtol = 100.0 KJ/mol/nm

Step 2 MD equilibration

Conditions: NVT ensemble (Nose-Hoover method),
temperature: 300 K, computation time: 10 (ps)
time step dt: 1 fs

Step 3 Production run

Conditions: NVT ensemble (Nose-Hoover method),
temperature: 300 K, computation time: 25,000 (ps)
time step dt: 1 fs

(5) Calculating the solvation free energy $\Delta\mu$ by the energy representation method

The solvation free energy $\Delta\mu$ of each of the monomer (a) alone, the PTFE crystal system alone, and the interface system between the monomer (a) and the PTFE is calculated by the energy representation method using the data obtained in (4) Molecular dynamics calculations. The calculation software used is ERmod (ver. 0.3.6) produced by Structural Chemistry Lab (Matsubayashi Group), Department of Molecular Chemistry, Graduate School of Engineering, Osaka University (or its equivalent). In (3) creating calculation systems, multiple initial configurations of the PTFE and the monomer (a) are used. The calculations are performed for each of the configurations, and the solvation free energy $\Delta\mu$ is calculated as the statistical average of the results.
In the compound of the present invention, the structural unit (A) may be in a side chain, in a backbone structure, or at a terminal of the backbone structure. In particular, in the compound of the present invention, the structural unit (A) is preferably in the backbone structure or at a terminal of the backbone structure so that the compound can have suitable physical properties necessary for use as a tackifier resin.
Specifically, for example, the structural unit (A) is preferably at least one selected from the group consisting of a structural unit (A-1) and a structural unit (A-1') that are represented by the following formulas.

[Chem. 1]

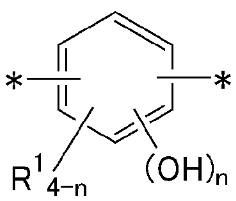 (A-1)

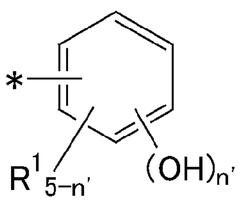 (A-1')

In the formulas, each $R^1$ represents a hydrogen atom, an aliphatic hydrocarbon group, an aromatic hydrocarbon group, a polar functional group, an aliphatic hydrocarbon group containing a polar functional group, or an aromatic hydrocarbon group containing a polar functional group; n represents an integer of 2 or greater and 4 or less; and n' represents an integer of 2 or greater and 5 or less. Each * represents a linking moiety.
In the structural unit (A-1) and the structural unit (A-1'), each $R^1$ represents a hydrogen atom, an aliphatic hydrocarbon group, an aromatic hydrocarbon group, a polar functional group, an aliphatic hydrocarbon group containing a polar functional group, or an aromatic hydrocarbon group containing a polar functional group.
The aliphatic hydrocarbon group is not limited. Examples thereof include C1-C20 linear, branched, or cyclic alkyl groups.
The aromatic hydrocarbon group is not limited. Examples thereof include substituted or non-substituted C1-C20 aryl groups.
The polar functional group is not limited. Examples thereof include amino, carboxy, carbonyl, alkoxy, hydroxy, nitrile, and nitro groups. The aliphatic hydrocarbon group containing a polar functional group is not limited and may be, for example, a group obtained by replacing one or more hydrogen atoms of any of the above aliphatic hydrocarbon groups with any of the above polar functional groups. The aromatic hydrocarbon group containing a polar functional group is not limited and may be, for example, a group obtained by replacing one or more hydrogen atoms of any of the above aromatic hydrocarbon groups with any of the above polar functional groups.
In the compound of the present invention, $R^1$s in one structural unit (A-1) may be the same as or different from each other, and $R^1$s in different structural units (A-1) may also be the same as or different from each other. Similarly, $R^1$s in one structural unit (A-1') may be the same as or different from each other, and $R^1$s in different structural units (A-1') may also be the same as or different from each other.

In the structural unit (A-1) and the structural unit (A-1'), n and n' are not limited as long as n is an integer of 2 or greater and 4 or less and n' is an integer of 2 or greater and 5 or less. For easy availability of raw materials, n and n' are preferably 2 or 3. More preferably, n and n' are 3 so that the compound can further increase the adhesion strength of adhesive compositions, particularly, even to low polarity adherends.

Specific examples of the structural unit (A-1) and the structural unit (A-1') include structural units derived from dihydroxybenzenes or their derivatives (n and n' are 2) and structural units derived from trihydroxybenzenes or their derivatives (n and n' are 3). These structural units may be used alone or in combination of two or more thereof.

The dihydroxybenzenes or their derivatives are not limited. Examples thereof include resorcinol, pyrocatechol, hydroquinone, dihydroxytoluene, dihydroxyxylene, dihydroxyphenyl ethylamine hydrochloride, dihydroxybenzoic acid, dihydroxyphenylacetic acid, dihydroxyhydrocinnamic acid, dihydroxyphenylpropionic acid, dihydroxyphenylalanine, dihydroxybenzaldehyde, dihydroxyacetophenone, diacetyldihydroxybenzene, dihydroxyphenyl-2-butanone, methyl dihydroxyphenyl acetate, benzyl dihydroxyphenyl ketone, dihydroxybenzamide, dihydroxymethoxybenzene, dihydroxybenzyl alcohol, dihydroxyphenyl ethanol, dihydroxyphenyl glycol, dihydroxyphenyl acetonitrile, and dihydroxynitrobenzene. These dihydroxybenzenes or their derivatives may be used alone or in combination of two or more thereof. Preferred among these is pyrocatechol, which has less steric hindrance and easily interacts with adherends.

The trihydroxybenzenes or their derivatives are not limited. Examples thereof include pyrogallol, 1,2,4-trihydroxybenzene, phloroglucinol, trihydroxytoluene, trihydroxydiphenylmethane, 6-hydroxy-L-DOPA, gallic acid, methyl gallate, butyl gallate, isobutyl gallate, isoamyl gallate, hexadecyl gallate, stearyl gallate, trihydroxyacetophenone, trihydroxyphenylethanone, trihydroxyphenylbutanone, trihydroxybenzaldehyde, trihydroxybenzamide, and trihydroxynitrobenzene. These trihydroxybenzenes or their derivatives may be used alone or in combination of two or more thereof. Preferred among these is pyrogallol, which has less steric hindrance and easily interacts with adherends.

The structural unit (A) is also preferably at least one selected from the group consisting of a structural unit (A-2), a structural unit (A-2'), a structural unit (A-3), a structural unit (A-3'), a structural unit (A-4), and a structural unit (A-4') that are represented by the following formulas.

[Chem. 2]

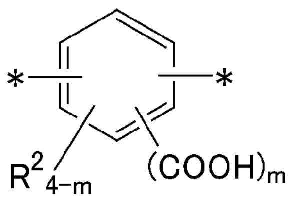

(A-2)

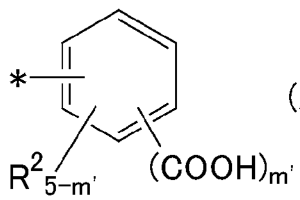

(A-2')

[Chem. 3]

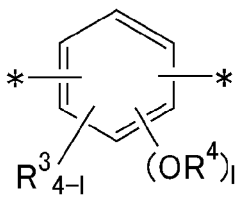

(A-3)

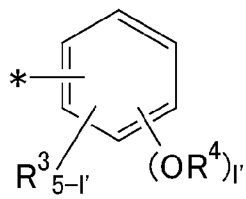

(A-3')

[Chem. 4]

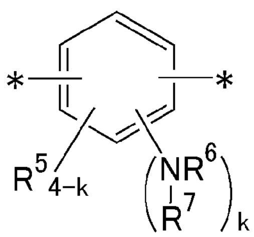

(A-4)

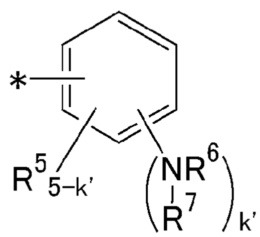

(A-4')

In the formulas, each $R^2$, each $R^3$, and each $R^5$ represents a hydrogen atom, an aliphatic hydrocarbon group, an aromatic hydrocarbon group, a polar functional group, an aliphatic hydrocarbon group containing a polar functional group, or an aromatic hydrocarbon group containing a polar functional group; each $R^4$ represents an aliphatic hydrocarbon group, an aromatic hydrocarbon group, an aliphatic hydrocarbon group containing a polar functional group, or an aromatic hydrocarbon group containing a polar functional group; each $R^6$ and each $R^7$ represents a hydrogen atom or an aliphatic hydrocarbon group; m represents an integer of 1 or greater and 4 or less; m' represents an integer of 1 or greater and 5 or less; l represents an integer of 2 or greater and 4 or less; l' represents an integer of 2 or greater and 5 or less; k represents an integer of 1 or greater and 4 or less; and k' represents an integer of 1 or greater and 5 or less. Each * represents a linking moiety.

In the structural unit (A-2) and the structural unit (A-2'), each $R^2$ represents a hydrogen atom, an aliphatic hydrocarbon group, an aromatic hydrocarbon group, a polar functional group, an aliphatic hydrocarbon group containing a polar functional group, or an aromatic hydrocarbon group containing a polar functional group.

The aliphatic hydrocarbon group is not limited. Examples thereof include C1-C20 linear, branched, or cyclic alkyl groups. The aromatic hydrocarbon group is not limited. Examples thereof include substituted or non-substituted C1-C20 aryl groups.

The polar functional group is not limited. Examples thereof include amino, carbonyl, alkoxy, hydroxy, nitrile, and nitro groups.

The aliphatic hydrocarbon group containing a polar functional group is not limited, and may be, for example, a group obtained by replacing one or more hydrogen atoms of any of the above aliphatic hydrocarbon groups with any of the above polar functional groups. The aromatic hydrocarbon group containing a polar functional group is not limited and may be, for example, a group obtained by replacing one or more hydrogen atoms of any of the above aromatic hydrocarbon groups with any of the above polar functional groups.

In the compound of the present invention, $R^2$s in one structural unit (A-2) may be the same as or different from each other, and $R^2$s in different structural units (A-2) may also be the same as or different from each other. Similarly, $R^2$s in one structural unit (A-2') may be the same as or different from each other, and $R^2$s in different structural units (A-2') may also be the same as or different from each other.

In the structural unit (A-2) and the structural unit (A-2'), m and m' are not limited as long as m is an integer of 1 or greater and 4 or less and m' is an integer of 1 or greater and 5 or less. For easy availability of raw materials, m and m' are preferably 1 or 2. More preferably, m and m' are 1 so that the compound can further increase the adhesion strength of adhesive compositions, particularly, even to low polarity adherends.

Specific examples of the structural unit (A-2) and the structural unit (A-2') include structural units derived from benzoic acid, salicylic acid, dihydroxybenzoic acid, gallic acid, 2-methylbenzoic acid, 3-methylbenzoic acid, 4-methylbenzoic acid, 2-ethylbenzoic acid, 3-ethylbenzoic acid, 4-ethylbenzoic acid, 4-tert-butylbenzoic acid, 2-vinylbenzoic acid, 3-vinylbenzoic acid, 4-vinylbenzoic acid, and derivatives thereof. These structural units may be used alone or in combination of two or more thereof. Preferred among these is 4-vinylbenzoic acid, which has less steric hindrance and easily interacts with adherends.

In the structural unit (A-3) and the structural unit (A-3'), each $R^3$ represents a hydrogen atom, an aliphatic hydrocarbon group, an aromatic hydrocarbon group, a polar functional group, an aliphatic hydrocarbon group containing a polar functional group, or an aromatic hydrocarbon group containing a polar functional group.

The aliphatic hydrocarbon group is not limited. Examples thereof include C1-C20 linear, branched, or cyclic alkyl groups. The aromatic hydrocarbon group is not limited. Examples thereof include substituted or non-substituted C1-C20 aryl groups.

The polar functional group is not limited. Examples thereof include amino, carboxy, carbonyl, hydroxy, nitrile, and nitro groups.

The aliphatic hydrocarbon group containing a polar functional group is not limited, and may be, for example, a group obtained by replacing one or more hydrogen atoms of any of the above aliphatic hydrocarbon groups with any of the above polar functional groups. The aromatic hydrocarbon group containing a polar functional group is not limited and may be, for example, a group obtained by replacing one or more hydrogen atoms of any of the above aromatic hydrocarbon groups with any of the above polar functional groups.

In the compound of the present invention, $R^3$s in one structural unit (A-3) may be the same as or different from each other, and $R^3$s in different structural units (A-3) may also be the same as or different from each other. Similarly, $R^3$s in one structural unit (A-3') may be the same as or different from each other, and $R^3$s in different structural units (A-3') may also be the same as or different from each other.

In the structural unit (A-3) and the structural unit (A-3'), each $R^4$ represents an aliphatic hydrocarbon group, an aromatic hydrocarbon group, an aliphatic hydrocarbon group containing a polar functional group, or an aromatic hydrocarbon group containing a polar functional group.

The aliphatic hydrocarbon group is not limited. Examples thereof include C1-C20 linear, branched, or cyclic alkyl groups. The aromatic hydrocarbon group is not limited. Examples thereof include substituted or non-substituted

C1-C20 aryl groups. In the compound of the present invention, $R^4$s in one structural unit (A-3) may be the same as or different from each other, and $R^4$s in different structural units (A-3) may also be the same as or different from each other. Similarly, $R^4$s in one structural unit (A-3') may be the same as or different from each other, and $R^4$s in different structural units (A-3') may also be the same as or different from each other.

In the structural unit (A-3) and the structural unit (A-3'), l and l' are not limited as long as l is an integer of 2 or greater and 4 or less and l' is an integer of 2 or greater and 5 or less. For easy availability of raw materials, l and l' are preferably 2 or 3. More preferably, l and l' are 3 so that the compound can further increase the adhesion strength of adhesive compositions, particularly, even to low polarity adherends.

Specific examples of the structural unit (A-3) and the structural unit (A-3') include structural units derived from trialkoxybenzenes or their derivatives (l is 3). The trialkoxybenzenes or their derivatives are not limited. Examples thereof include 1,2,3-trimethoxybenzene, 1,2,4-trimethoxybenzene, and 1,3,5-trimethoxybenzene. These trialkoxybenzenes or their derivatives may be used alone or in combination of two or more thereof. Preferred among these is 1,2,3-trimethoxybenzene, which has less steric hindrance and easily interacts with adherends.

In the structural unit (A-4) and the structural unit (A-4'), each $R^5$ represents a hydrogen atom, an aliphatic hydrocarbon group, an aromatic hydrocarbon group, a polar functional group, an aliphatic hydrocarbon group containing a polar functional group, or an aromatic hydrocarbon group containing a polar functional group.

The aliphatic hydrocarbon group is not limited. Examples thereof include C1-C20 linear, branched, or cyclic alkyl groups. The aromatic hydrocarbon group is not limited. Examples thereof include substituted or non-substituted C1-C20 aryl groups.

The polar functional group is not limited. Examples thereof include carboxy, carbonyl, alkoxy, hydroxy, nitrile, and nitro groups.

The aliphatic hydrocarbon group containing a polar functional group is not limited, and may be, for example, a group obtained by replacing one or more hydrogen atoms of any of the above aliphatic hydrocarbon groups with any of the above polar functional groups. The aromatic hydrocarbon group containing a polar functional group is not limited and may be, for example, a group obtained by replacing one or more hydrogen atoms of any of the above aromatic hydrocarbon groups with any of the above polar functional groups.

In the compound of the present invention, $R^5$s in one structural unit (A-4) may be the same as or different from each other, and $R^5$s in different structural units (A-4) may also be the same as or different from each other. Similarly, $R^5$s in one structural unit (A-4') may be the same as or different from each other, and $R^5$s in different structural units (A-4') may also be the same as or different from each other.

In the structural unit (A-4) and the structural unit (A-4'), each $R^6$ and each $R^7$ represents a hydrogen atom or an aliphatic hydrocarbon group.

The aliphatic hydrocarbon group is not limited. Examples thereof include C1-C20 linear, branched, or cyclic alkyl groups. In the compound of the present invention, $R^6$s and $R^7$s in one structural unit (A-4) may be the same as or different from each other, and $R^6$s and $R^7$s in different structural units (A-4) may be the same as or different from each other. Similarly, $R^6$s and $R^7$s in one structural unit (A-4') may be the same as or different from each other, and $R^6$s and $R^7$s in different structural units (A-4') may be the same as or different from each other.

In the structural unit (A-4) and the structural unit (A-4'), k and k' are not limited as long as k is an integer of 1 or greater and 4 or less and k' is an integer of 1 or greater and 5 or less. For easy availability of raw materials, k and k' are preferably 1, 2, or 3. More preferably, k and k' are 1 so that the compound can further increase the adhesion strength of adhesive compositions, particularly, even to low polarity adherends.

Specific examples of the structural unit (A-4) and the structural unit (A-4') include structural units derived from aminobenzene or its derivatives (k is 1). The aminobenzene or its derivatives are not limited. Examples thereof include aniline, methylaniline, ethylaniline, dimethylaniline, and diethylaniline. The aminobenzene or its derivatives may be used alone or in combination of two or more thereof.

The structural unit (A) may consist only of a petroleum-derived material, but preferably contains a bio-derived material. As the depletion of petroleum resources and carbon oxide emissions from the combustion of petroleum-derived products have become problematic, attempts have been made to save petroleum resources by using bio-derived materials instead of petroleum-derived materials. The structural unit (A) containing a bio-derived material is preferable to save petroleum resources. The structural unit (A) containing a bio-derived material is also preferable to reduce carbon dioxide emissions because combusting bio-derived materials, which originally form by incorporating atmospheric carbon dioxide, is not considered to increase the total amount of atmospheric carbon dioxide.

Examples of a monomer that constitutes the structural unit (A) containing a bio-derived material include resorcinol, dihydroxyphenyl ethylamine hydrochloride, dihydroxyhydrocinnamic acid, dihydroxyphenylalanine, dihydroxybenzaldehyde, dihydroxybenzyl alcohol, pyrogallol, 1,2,4-trihydroxybenzene, phloroglucinol, 6-hydroxy-L-DOPA, gallic acid, methyl gallate, butyl gallate, isobutyl gallate, isoamyl gallate, hexadecyl gallate, stearyl gallate, trihydroxyacetophenone, trihydroxybenzaldehyde, trihydroxybenzamide, and trihydroxynitrobenzene.

The structural unit (A) content of the compound of the present invention is not limited. The lower limit thereof

is preferably 1 mol%, and the upper limit thereof is preferably 60 mol%. When the structural unit (A) content is 1 mol% or greater, adding the compound to an adhesive composition can further increase the adhesion strength of the adhesive composition, particularly, even to low polarity adherends. When the structural unit (A) content is 60 mol% or less, the compound can have suitable physical properties required for use as a tackifier resin. The lower limit of the structural unit (A) content is more preferably 5 mol%, and the upper limit thereof is more preferably 50 mol%. The lower limit is still more preferably 10 mol%, and the upper limit is still more preferably 30 mol%.

The compound of the present invention includes a structural unit (B) derived from at least one monomer (b) selected from the group consisting of a terpene monomer, a vinyl monomer, and a conjugated diene monomer. In other words, the compound of the present invention further includes, in addition to the structural unit (A), a structural unit (B) derived from at least one monomer (b) selected from the group consisting of a terpene monomer, a vinyl monomer, and a conjugated diene monomer. The presence of the structural unit (B) allows the compound to have suitable physical properties required for use as a tackifier resin.

In particular, the structural unit (B) is preferably a structural unit derived from a terpene monomer or a structural unit derived from a vinyl monomer, or also preferably a combination of a structural unit derived from a terpene monomer and a structural unit derived from a vinyl monomer so that adding the compound to an adhesive composition can further increase the adhesion strength of the adhesive composition. To improve the compatibility of the compound with base polymers, particularly with styrene elastomers, the structural unit (B) is preferably a structural unit derived from a terpene monomer or a structural unit derived from a conjugated diene monomer. These structural units contain an aliphatic hydrocarbon group containing an unsaturated double bond. Thus, the compound including any of these structural units can have improved compatibility with base polymers, particularly with styrene elastomers, and can prevent or reduce a decrease in the adhesion strength of the adhesive composition due to reduced compatibility.

The terpene monomer is not limited. Examples thereof include $\alpha$-pinene, $\beta$-pinene, limonene, dipentene, $\delta$-3-carene, dimethyl octatriene, allo-ocimene, myrcene, ocimene, linalool, and cosmene. In particular, $\alpha$-pinene, $\beta$-pinene, and limonene are preferred so that adding the compound to an adhesive composition can further increase the adhesion strength of the adhesive composition.

The vinyl monomer is not limited. To improve the compatibility of the compound with base polymers, particularly with acrylic polymers, the vinyl monomer is preferably free of a structure containing two or more aromatic rings in a molecule (e.g., a naphthalene structure, an anthracene structure, a biphenyl structure, an anthraquinone structure, a benzophenone structure). Examples of vinyl monomers free of a structure containing two or more aromatic rings in a molecule include ethylene, propylene, butylene, hexene, vinyl acetate, vinyl chloride, styrene, $\alpha$-methylstyrene, coumarone, indene, vinyl toluene, divinylbenzene, divinyl toluene, and 2-phenyl-2-butene. In particular, styrene is preferred so that adding the compound to an adhesive composition can further increase the adhesion strength of the adhesive composition.

The conjugated diene monomer is not limited. Examples thereof include butadiene, isoprene, piperylene, and cyclopentadiene. In particular, isoprene is preferred for further increase in the adhesion strength of an adhesive composition by adding the compound to the adhesive composition.

These monomers (b) may be used alone or in combination of two or more thereof.

The structural unit (B) may consist only of a petroleum-derived material but preferably contains a bio-derived material. As the depletion of petroleum resources and carbon oxide emissions from the combustion of petroleum-derived products have become problematic, attempts have been made to save petroleum resources by using bio-derived materials instead of petroleum-derived materials. The structural unit (B) containing a bio-derived material is preferable to save petroleum resources. The structural unit (B) containing a bio-derived material is also preferable to reduce carbon dioxide emissions because combusting bio-derived materials, which originally form by incorporating atmospheric carbon dioxide, is not considered to increase the total amount of atmospheric carbon dioxide.

Examples of the monomer (b) that constitutes the structural unit (B) containing a bio-derived material include terpene monomers, ethylene, propylene, hexene, butadiene, and isoprene.

The structural unit (B) content of the compound of the present invention is not limited. The lower limit thereof is preferably 40 mol%, and the upper limit thereof is preferably 99 mol%. When the structural unit (B) content is 40 mol% or greater, the compound can have suitable physical properties required for use as a tackifier resin. When the structural unit (B) content is 99 mol% or less, the compound can have a sufficient structural unit (A) content, so that adding the compound to an adhesive composition can further increase the adhesion strength of the adhesive composition, particularly, even to low polarity adherends. The lower limit of the structural unit (B) content is more preferably 50 mol%, and the upper limit thereof is more preferably 90 mol%.

The compound of the present invention may be any compound that contains the structural unit (A) and the structural unit (B), but is preferably a copolymer having a structure represented by the formula below.

A copolymer having such a structure can be obtained by a method that uses cationic polymerization described later. The copolymer can further increase the adhesion strength of adhesive compositions, particularly, even to low polarity adherends.

[Chem. 5]

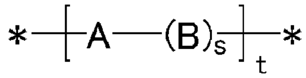

In the formula, A represents the structural unit (A); B represents the structural unit (B); and s and t each represent an integer of 1 or greater. Each * represents a linking moiety.

The compound of the present invention may be any compound including the structural unit (A) and the structural unit (B). The compound is preferably a copolymer including the structural unit (A) and the structural unit (B) and may further include a different structural unit. In the copolymer, the structural unit (A) and the structural unit (B) may be randomly copolymerized, or may be regularly or periodically copolymerized as in the case where, for example, they each form a block segment and the block segments bond to each other.

The compound of the present invention preferably contains an aliphatic hydrocarbon group containing an unsaturated double bond.

In the compound of the present invention, the aliphatic hydrocarbon group containing an unsaturated double bond may be in the structural unit (A) or the structural unit (B) or may be in the different structural unit. In particular, for ease of synthesis and improvement in the compatibility of the compound with base polymers, particularly with styrene elastomers, the aliphatic hydrocarbon group containing an unsaturated double bond is preferably in the structural unit (B) or the different structural unit. Such a structural unit (B) or different structural unit containing the aliphatic hydrocarbon group containing an unsaturated double bond is not limited. Preferred is a structural unit (B) derived from at least one monomer (b) selected from the group consisting of a terpene monomer and a conjugated diene monomer. In other words, in the compound of the present invention, the aliphatic hydrocarbon group containing an unsaturated double bond is preferably in a structural unit (B) derived from at least one monomer (b) selected from the group consisting of a terpene monomer and a conjugated diene monomer. In particular, the aliphatic hydrocarbon group containing an unsaturated double bond is preferably in a structural unit derived from a terpene monomer so that adding the compound to an adhesive composition can further increase the adhesion strength of the adhesive composition.

Examples of the different structural unit also include: a structural unit derived from a different phenol monomer and not encompassed by the structural unit (A); and a structural unit derived from maleic anhydride.

The different phenol monomer is not limited. Examples thereof include phenol, cresol, xylenol, propyl phenol, nonyl phenol, methoxy phenol, bromophenol, bisphenol A, bisphenol F, bisphenol S, and dihydroxynaphthalene. These different phenol monomers may be used alone or in combination of two or more thereof.

The molecular weight of the compound of the present invention is not limited. The lower limit of the weight average molecular weight (Mw) is preferably 400, and the upper limit thereof is preferably 10,000. When the weight average molecular weight (Mw) is within the above range, the compound can have suitable physical properties required for use as a tackifier resin. The lower limit of the weight average molecular weight (Mw) is more preferably 500, and the upper limit thereof is more preferably 5,000. The lower limit is still more preferably 700, and the upper limit is still more preferably 3,000.

The weight average molecular weight (Mw) may be adjusted to the above range by, for example, adjusting the composition, polymerization method, and polymerization conditions of the compound.

The weight average molecular weight (Mw) and the later-described molecular weight distribution (Mw/Mn) can be measured by the following method.

A solution of the compound is filtered through a filter (material: polytetrafluoroethylene, pore size: 0.2 um). The obtained filtrate is supplied to a gel permeation chromatograph (e.g., 2690 Separations Model, produced by Waters) and subjected to GPC measurement at a sample flow rate of 1 mL/min and a column temperature of 40°C. The polystyrene equivalent molecular weight of the compound is thus measured, and the weight average molecular weight (Mw) and the molecular weight distribution (Mw/Mn) are determined. A column used is GPC KF-802.5L (produced by Showa Denko K.K.). A detector used is a differential refractometer.

The Young's modulus of the compound of the present invention is not limited. The lower limit of the Young's modulus at 25°C is preferably 10 MPa. When the Young's modulus at 25°C is 10 MPa or greater, the compound can have appropriate hardness and have suitable physical properties required for use as a tackifier resin, rather than as an adhesive. The lower limit of the Young's modulus at 25°C is more preferably 50 MPa, still more preferably 70 MPa.

The upper limit of the Young's modulus at 25°C is not limited. To prevent or reduce a decrease in the adhesion strength due to excessive hardness of the adhesive composition containing the compound, the upper limit is preferably 10,000 MPa, more preferably 5,000 MPa.

The Young's modulus at 25°C may be adjusted to the above range by, for example, adjusting the molecular weight of the compound, the compositions of the structural unit (A) and the structural unit (B) in the compound, and the structural unit (A) and structural unit (B) contents.

The Young's modulus at 25°C can be measured using a tensile tester (e.g., TENSILON, produced by ORIENTEC) by a tensile test at a tensile speed of 200 mm/min, a clamp distance of 15 mm, and 25°C. The measurement sample for the test may be prepared by, for example, filling a mold having a size of 10 × 50 mm with the compound and melting the compound at a temperature 100°C higher than the glass transition temperature of the compound to form a specimen having a thickness of 1 mm.

The glass transition temperature of the compound of the present invention is not limited. The lower limit thereof is preferably 0°C, and the upper limit thereof is preferably 200°C. When the glass transition temperature is within the range, the Young's modulus of the compound can be easily adjusted to the above range, allowing the compound to have suitable physical properties required for use as a tackifier resin. The lower limit of the glass transition temperature is more preferably 10°C, and the upper limit thereof is more preferably 150°C.

The glass transition temperature can be measured using a differential scanning calorimeter (e.g., SII Exstar 6000/DSC 6220, produced by Hitachi High-Tech Science Corporation) in a nitrogen atmosphere at a heating rate of 10°C/min. The value obtained in the first run can be used as the glass transition temperature.

The iodine value of the compound of the present invention is not limited. The lower limit thereof is preferably 2 g/100 g, and the upper limit thereof is preferably 180 g/100 g. When the iodine value is 2 g/100 g or greater, a decrease in the adhesion strength of an adhesive composition due to reduced compatibility of the compound with base polymers, particularly with styrene elastomers, can be prevented or reduced. When the iodine value is 180 g/100 g or less, adding the compound to an adhesive composition can further increase the adhesion strength of the adhesive composition, particularly, even to low polarity adherends. The lower limit of the iodine value is more preferably 70 g/100 g, and the upper limit thereof is more preferably 170 g/100 g.

The iodine value is an index of the amount of unsaturated double bonds (C=C bond content) and herein refers to an iodine value measured in conformity with the method described in JIS K 0070:1992.

The bio-derived carbon (carbon atoms) content in the carbon (carbon atoms) in the compound of the present invention is not limited. The bio-derived carbon content in the carbon in the compound is preferably 10% or greater. A bio-derived carbon content of 10% or greater is an indicator of a "bio-based product".

A bio-derived carbon content of 10% or greater is preferred to save petroleum resources and reduce carbon dioxide emissions. The lower limit of the bio-derived carbon content is more preferably 30%, still more preferably 60%, further preferably 70%, still further preferably 90%. The upper limit of the bio-derived carbon content is not limited and may be 100%.

While bio-derived carbon contains a certain proportion of radioisotope (C-14), petroleum-derived carbon hardly contains C-14. Thus, the bio-derived carbon content can be calculated by measuring the C-14 concentration in the compound. Specifically, the bio-derived carbon content can be measured in conformity with ASTM D6866-20, a standard widely used in the bioplastics industry.

The compound of the present invention encompasses a hydrogenated product of the compound described above. The hydrogenated product is a compound obtained by saturating a carbon-carbon double bond in the compound described above by hydrogenation. The hydrogenated product also can be suitably used as a tackifier resin to add to adhesive compositions and can increase the adhesion strength of adhesive compositions, particularly, even to low polarity adherends.

The compound of the present invention may be produced by any method. For example, the following method is preferred: a method for producing a compound including a structural unit (A) derived from a monomer (a) having a solvation free energy $\Delta\mu$ with polytetrafluoroethylene of - 30 kcal/mol or less and a structural unit (B) derived from at least one monomer (b) selected from the group consisting of a terpene monomer, a vinyl monomer, and a conjugated diene monomer, the method including copolymerizing the monomer (a) and the monomer (b). The present invention also encompasses such a method for producing a compound.

The monomer (a) may be any monomer having a solvation free energy $\Delta\mu$ with polytetrafluoroethylene of -30 kcal/mol or less. The monomer (a) is preferably at least one selected from the group consisting of a monomer (a-1), a monomer (a-2), a monomer (a-3), and a monomer (a-4) that are represented by the following formulas.

[Chem. 6]

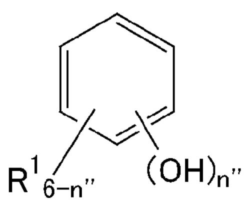

(a-1)

In the formula, each $R^1$ represents a hydrogen atom, an aliphatic hydrocarbon group, an aromatic hydrocarbon group, a polar functional group, an aliphatic hydrocarbon group containing a polar functional group, or an aromatic hydrocarbon group containing a polar functional group; and n" represents an integer of 2 or greater and 5 or less.
n" is preferably 2 or 3, more preferably 3.

[Chem. 7]

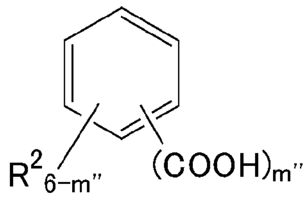

(a-2)

[Chem. 8]

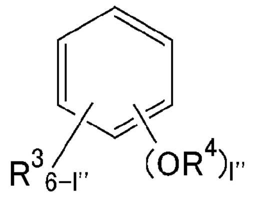

(a-3)

[Chem. 9]

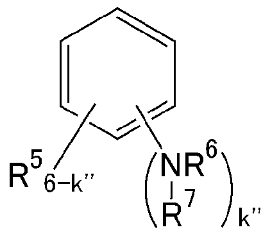

(a-4)

In the formulas, each $R^2$, each $R^3$, and each $R^5$ represents a hydrogen atom, an aliphatic hydrocarbon group, an aromatic hydrocarbon group, a polar functional group, an aliphatic hydrocarbon group containing a polar functional group, or an aromatic hydrocarbon group containing a polar functional group; each $R^4$ represents an aliphatic hydrocarbon group, an aromatic hydrocarbon group, an aliphatic hydrocarbon group containing a polar functional group, or an aromatic hydrocarbon group containing a polar functional group; each $R^6$ and each $R^7$ represents a hydrogen atom or an aliphatic hydrocarbon group; m" represents an integer of 1 or greater and 5 or less; 1" represents an integer of 2 or greater and 5 or less; and k" represents an integer of 1 or greater and 5 or less.
In the method for producing a compound of the present invention, the monomer (a) and the monomer (b) are preferably copolymerized by cationic polymerization.
Cationic polymerization enables copolymerization of the monomer (a) and the monomer (b) without protecting functional groups of the monomer (a) such as phenolic hydroxy groups, carboxy groups, alkoxy groups, and amino groups by chemical modification in advance, thus eliminating the need for later deprotection. The monomer (a) and the monomer (b) thus can be copolymerized by a simpler, one-stage reaction process, leading to fewer impurities and higher yield.
The monomer (a) and the monomer (b) are cationically copolymerized preferably by a method including reacting the monomer (a) and the monomer (b) in the presence of a Lewis acid. With such a method, a cation of the monomer (b) is considered to be formed and cause cationic polymerization of the molecules of the monomer (b) to proceed while Fridel-Crafts alkylation of the monomer (a) and the monomer (b) proceeds. These reactions repeat to produce the copolymer including the structural unit (A) derived from the monomer (a) and the structural unit (B) derived from the monomer (b).
The Lewis acid is not limited. A conventionally known Lewis acid may be used. Examples thereof include aluminum chloride ($AlCl_3$), diethylaluminum chloride ($Et_2AlCl_2$), tin(IV) chloride ($SnCl_4$), titanium(IV) chloride ($TiCl_4$),

boron trichloride ($BCl_3$), and a boron trifluoride ether complex ($BF_3 \cdot EtO$). Preferred among these is aluminum chloride ($AlCl_3$), which enables higher yield.

More specifically, for example, when pyrogallol as the monomer (a) and α-pinene as the monomer (b) are reacted in the presence of aluminum chloride ($AlCl_3$), a Lewis acid, the reaction shown in the scheme below is considered to proceed.

Specifically, a cation of α-pinene as the monomer (b) is formed and causes cationic polymerization of molecules of α-pinene to proceed (the upper section of the scheme below) while Fridel-Crafts alkylation of pyrogallol as the monomer (a) and α-pinene as the monomer (b) proceeds (the middle section of the scheme below). These reactions repeat to produce a copolymer including a structural unit derived from pyrogallol and a structural unit derived from α-pinene (the lower section of the scheme below). Here, in such a copolymer, the structural unit derived from pyrogallol is in the backbone structure or at a terminal of the backbone structure.

[Chem. 10]

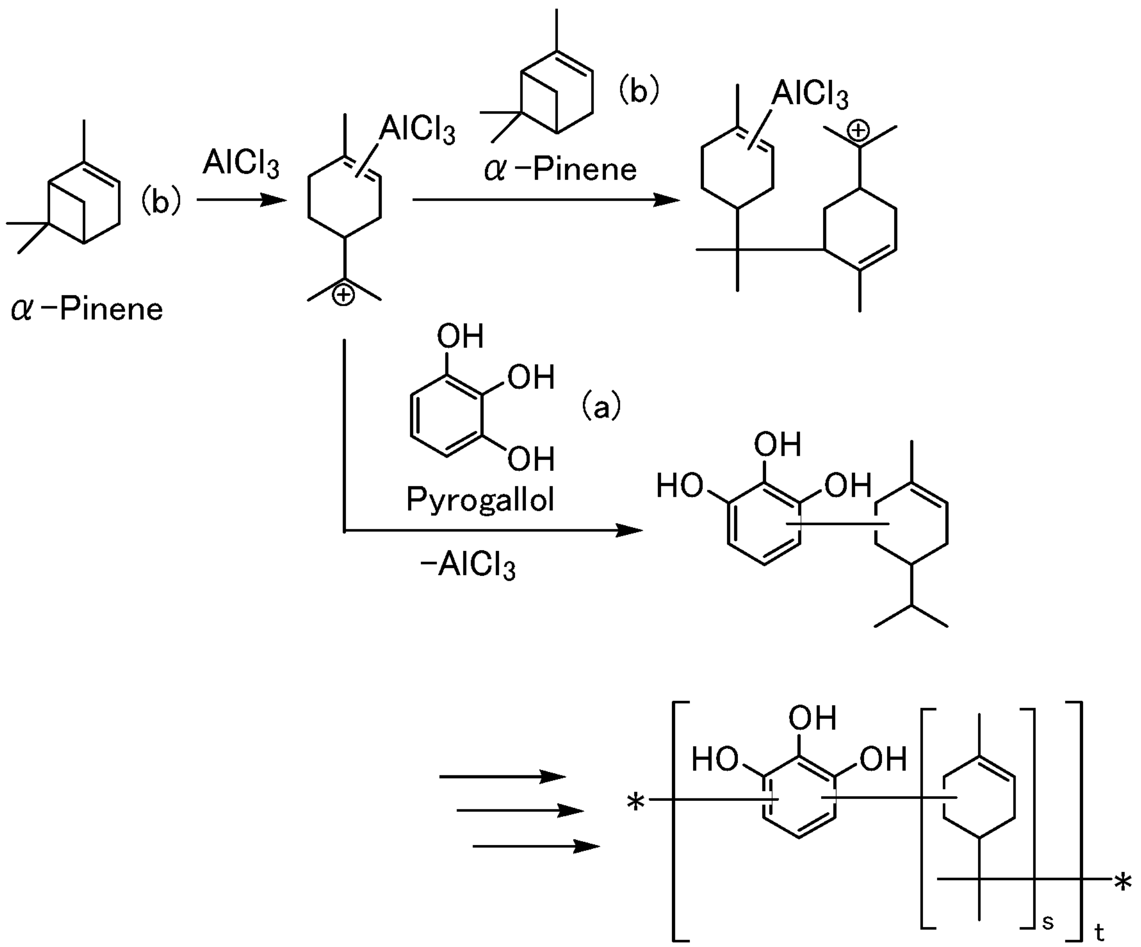


In the formula, s and t each represent an integer of 1 or greater. Each * represents a linking moiety.

The compound of the present invention can be suitable as a tackifier resin to add to adhesive compositions. The present invention also encompasses an adhesive composition containing a base polymer and the compound (T1) of the present invention.

The amount of the compound (T1) of the present invention in the adhesive composition of the present invention is not limited. Even in a smaller amount than conventional tackifier resins, the compound (T1) can increase the adhesion strength of the adhesive composition. The lower limit of the amount of the compound (T1) relative to 100 parts by weight of the base polymer is preferably 1 part by weight, and the upper limit thereof is preferably 35 parts by weight. When the amount of the compound (T1) of the present invention is 1 part by weight or greater, the compound (T1) can further increase the adhesion strength of the adhesive composition, particularly, even to low polarity adherends. When the amount of the compound (T1) of the present invention is 35 parts by weight or less, a decrease in the adhesion strength due to excessive hardness of the adhesive composition can be prevented or reduced. The lower limit of the amount of the compound (T1) of the present invention is more preferably 3 parts by weight, and the upper limit thereof is more

preferably 30 parts by weight. The lower limit is still more preferably 5 parts by weight, and the upper limit is still more preferably 20 parts by weight.

The adhesive composition of the present invention may further contain at least one tackifier resin (T2) selected from the group consisting of a rosin ester resin, a terpene resin, and a petroleum resin. In particular, a rosin ester resin or a terpene resin is preferred so that the adhesion strength of the adhesive composition can be further increased.

The lower limit of the softening temperature of the tackifier resin (T2) is preferably 70°C, and the upper limit thereof is preferably 170°C. When the softening temperature is 70°C or higher, a decrease in the adhesion strength due to excessive softness of the adhesive composition can be prevented or reduced. When the softening temperature is 170°C or lower, an adhesive layer formed from the adhesive composition can have improved interfacial wettability and can be less prone to interfacial peeling. The lower limit of the softening temperature is more preferably 120°C.

The softening temperature is measured by the ring and ball method specified in JIS K2207.

The lower limit of the hydroxy value of the tackifier resin (T2) is preferably 25, and the upper limit thereof is preferably 150. When the hydroxy value is within the above range, an adhesive layer formed from the adhesive composition can have improved interfacial wettability and can be less prone to interfacial peeling. The lower limit of the hydroxy value is more preferably 30, and the upper limit thereof is more preferably 130.

The hydroxy value can be measured in conformity with JIS K1557 (phthalic anhydride method).

The amount of the tackifier resin (T2) is not limited. The lower limit of the amount relative to 100 parts by weight of the base polymer is preferably 10 parts by weight, and the upper limit thereof is preferably 100 parts by weight. When the amount of the tackifier resin (T2) is 10 parts by weight or greater, the adhesive composition can have higher adhesion strength. When the amount of the tackifier resin (T2) is 100 parts by weight or less, a decrease in the adhesion strength due to excessive hardness of the adhesive composition can be prevented or reduced. The lower limit of the amount of the tackifier resin (T2) is more preferably 15 parts by weight, and the upper limit thereof is more preferably 60 parts by weight, still more preferably 50 parts by weight, further preferably 40 parts by weight.

The base polymer is not limited. Examples thereof include acrylic polymers, rubber polymers, urethane polymers, and silicone polymers. Preferably, the base polymer is an acrylic polymer because acrylic polymers are relatively stable to light, heat, moisture, and the like. Also preferably, the base polymer is a rubber polymer because rubber polymers, having low adherend selectivity, can adhere to various adherends and are less likely to peel off from adherends when immersed in an alkaline chemical solution. More preferred among the rubber polymers are styrene elastomers that are block copolymers including a block derived from a styrene monomer and a block derived from a conjugated diene monomer or hydrogenated products thereof.

To improve the initial tackiness for easier bonding at low temperature, the acrylic polymer preferably includes a structural unit derived from at least one selected from the group consisting of an alkyl (meth)acrylate containing a C1-C12 alkyl group and an alkyl (meth)acrylate containing a C13-C18 alkyl group.

Examples of the alkyl (meth)acrylate containing a C1-C12 alkyl group include 2-ethylhexyl (meth)acrylate, butyl (meth)acrylate, methyl (meth)acrylate, ethyl (meth)acrylate, n-propyl (meth)acrylate, and isopropyl (meth)acrylate. Examples of the alkyl (meth)acrylate containing a C13-C18 alkyl group include tridecyl methacrylate and stearyl (meth)acrylate. In particular, 2-ethylhexyl (meth)acrylate or butyl (meth)acrylate is preferably used so that the acrylic polymer can exhibit high adhesive force.

In the acrylic polymer, the amount of the structural unit derived from at least one selected from the group consisting of an alkyl (meth)acrylate containing a C1-C12 alkyl group and an alkyl (meth)acrylate containing a C13-C18 alkyl group is not limited. The lower limit of the amount is preferably 10% by weight, and the upper limit thereof is preferably 100% by weight. The lower limit is more preferably 30% by weight, and the upper limit is more preferably 95% by weight. The lower limit is still more preferably 50% by weight, and the upper limit is still more preferably 90% by weight. When the amount is within the range, the acrylic polymer can exhibit high adhesive force.

The acrylic polymer preferably includes a structural unit derived from a monomer containing a crosslinkable functional group.

When a crosslinking agent is added, the acrylic polymer including the structural unit derived from a monomer containing a crosslinkable functional group forms a crosslinked structure in an adhesive layer formed from the adhesive composition. This increases the gel fraction and the bulk strength of the adhesive layer, improving adhesion strength. The crosslinkable functional group is not limited. Examples thereof include amino, carboxy, carbonyl, hydroxy, epoxy, and isocyanate groups.

Specific examples of the monomer containing a crosslinkable functional group include hydroxyalkyl (meth)acrylate, glycerol dimethacrylate, glycidyl (meth)acrylate, 2-methacryloyloxyethyl isocyanate, (meth)acrylic acid, itaconic acid, maleic anhydride, crotonic acid, maleic acid, and fumaric acid. Specific examples of the hydroxyalkyl (meth)acrylate include 2-hydroxyethyl (meth)acrylate. These monomers containing a crosslinkable functional group may be used alone or in combination of two or more thereof. To increase the gel fraction and the bulk strength of the adhesive layer, monomers containing a hydroxy group, such as hydroxyalkyl (meth)acrylate and glycerol dimethacrylate, and monomers containing a carboxy (meth)acrylate group are preferred.

The amount of the structural unit derived from a monomer containing a crosslinkable functional group in the acrylic polymer is not limited. The lower limit thereof is preferably 0.01% by weight, and the upper limit thereof is preferably 20% by weight. When the amount of the structural unit derived from a monomer containing a crosslinkable functional group is within the range, an adhesive layer formed from the adhesive composition has higher gel fraction and higher bulk strength and thus has improved adhesion strength. The lower limit of the amount of the structural unit derived from a monomer containing a crosslinkable functional group is more preferably 0.05% by weight, and the upper limit thereof is more preferably 5% by weight.

The acrylic polymer may optionally contain a structural unit derived from a different copolymerizable monomer other than the above-described structural unit derived from an alkyl (meth)acrylate and the structural unit derived from a monomer containing a crosslinkable functional group.

The acrylic polymer can be obtained by radically reacting a mixture of the above monomers in the presence of a polymerization initiator. The method for radically reacting the monomer mixture, in other words, the polymerization method, may be a conventionally known method. Examples thereof include solution polymerization (boiling point polymerization or constant temperature polymerization), emulsion polymerization, suspension polymerization, and bulk polymerization.

The weight average molecular weight (Mw) of the acrylic polymer is not limited. The lower limit thereof is preferably 200,000, and the upper limit thereof is preferably 2,000,000. When the weight average molecular weight (Mw) is 200,000 or greater, an adhesive layer formed from the adhesive composition has higher bulk strength and thus has improved adhesion strength. When the weight average molecular weight (Mw) is 2,000,000 or less, an adhesive layer formed from the adhesive composition can have improved interfacial wettability and can be less prone to interfacial peeling. The lower limit of the weight average molecular weight (Mw) is more preferably 400,000, and the upper limit thereof is more preferably 1,500,000.

The ratio of the weight average molecular weight (Mw) to the number average molecular weight (Mn) of the acrylic polymer (molecular weight distribution, Mw/Mn) is not limited. The lower limit thereof is preferably 1.05, and the upper limit thereof is preferably 5.0. When the Mw/Mn is 5.0 or less, the acrylic polymer can have a reduced proportion of low molecular weight components. This increases the bulk strength of an adhesive layer formed from the adhesive composition, improving the adhesion strength. The upper limit of the Mw/Mn is more preferably 4.5, still more preferably 4, further preferably 3.5.

The weight average molecular weight (Mw) and the molecular weight distribution (Mw/Mn) may be adjusted to the above range by adjusting the composition, polymerization method, polymerization conditions of the acrylic polymer, for example.

The styrene elastomer may be any block copolymer that has rubber elasticity at room temperature and that includes a hard segment and a soft segment. Here, the block derived from a styrene monomer is a hard segment, and the block derived from a conjugated diene monomer is a soft segment.

The styrene monomer is not limited. Examples thereof include styrene, 2-methylstyrene, 3-methylstyrene, 4-methylstyrene, $\alpha$-methylstyrene, 2,4-dimethylstyrene, 2,4-diisopropylstyrene, 4-t-butylstyrene, 5-t-butyl-2-methylstyrene, vinylethylbenzene, divinylbenzene, trivinylbenzene, divinylnaphthalene, t-butoxystyrene, vinylbenzyldimethylamine, (4-vinylbenzyl)dimethylaminoethyl ether, N,N-dimethylaminoethylstyrene, N,N-dimethylaminomethylstyrene, 2-ethylstyrene, 3-ethylstyrene, 4-ethylstyrene, 2-t-butylstyrene, 3-t-butylstyrene, 4-t-butylstyrene, vinylxylene, vinylnaphthalene, vinylpyridine, diphenylethylene, and diphenylethylene containing a tertiary amino group. The diphenylethylene containing a tertiary amino group is not limited. Examples thereof include 1-(4-N,N-dimethylaminophenyl)-1-phenylethylene. These styrene monomers may be used alone or in combination of two or more thereof.

The conjugated diene monomer is not limited. Examples thereof include isoprene, 1,3-butadiene, 2,3-dimethyl-1,3-butadiene, 1,3-pentadiene, 1,3-hexadiene, 1,3-heptadiene, 2-phenyl-1,3-butadiene, 3-methyl-1,3-pentadiene, and 2-chloro-1,3-butadiene. These conjugated diene monomers may be used alone or in combination of two or more thereof.

Specific examples of the styrene elastomer include styrene-isoprene-styrene (SIS) block copolymers, styrenebutadiene-styrene (SBS) block copolymers, and styrene-chloroprene-styrene block copolymers. In particular, for the adhesive layer to easily exhibit high adhesion strength and to be less prone to peeling from adherends when immersed in an alkaline chemical solution, SIS block copolymers and SBS block copolymers are preferred, and SIS block copolymers are more preferred. These styrene elastomers may be used alone or in combination of two or more thereof.

The styrene elastomer may include a diblock copolymer of the block derived from a styrene monomer and the block derived from a conjugated diene monomer in addition to a triblock copolymer of the block derived from a styrene monomer and the block derived from a conjugated diene monomer.

The amount of the diblock copolymer (hereinafter also referred to as "diblock proportion") in the styrene elastomer is not limited. The lower limit thereof is preferably 50% by weight, more preferably 70% by weight. When the diblock proportion is within the range, the adhesive composition has high adhesion to adherends and improved adhesion strength. The upper limit of the diblock proportion is not limited. The upper limit is preferably 90% by weight to maintain the cohesive force of the adhesive composition.

The diblock proportion can be calculated from the peak area ratio of the copolymers measured by gel permeation chromatography (GPC).

The amount of the block derived from a styrene monomer (hereinafter also referred to as "styrene content") in the styrene elastomer is not limited. The upper limit thereof is preferably 20% by weight, more preferably 16% by weight. When the styrene content is within the above range, the adhesive composition is not too hard and has high adhesion to adherends and improved adhesion strength. The lower limit of the styrene content is not limited but is preferably 8% by weight to maintain the cohesive force of the adhesive composition.

The styrene content can be calculated from the peak area ratio of the blocks measured by $^1$H-NMR.

The weight average molecular weight of the styrene elastomer is not limited. The lower limit thereof is preferably 50,000, and the upper limit thereof is preferably 600,000. When the weight average molecular weight is 50,000 or greater, an adhesive layer formed from the adhesive composition has higher bulk strength and improved adhesion strength. When the weight average molecular weight is 600,000 or less, an excessive decrease in the compatibility of the styrene elastomer with other components can be prevented. The lower limit of the weight average molecular weight is more preferably 100,000, and the upper limit thereof is more preferably 500,000.

The adhesive composition of the present invention preferably contains a crosslinking agent when the base polymer is the acrylic polymer described above.

Adjusting the type and amount of the crosslinking agent enables easy adjustment of the gel fraction of an adhesive layer formed from the adhesive composition. The crosslinking agent is not limited. Examples thereof include isocyanate crosslinking agents, aziridine crosslinking agents, epoxy crosslinking agents, and metal chelate crosslinking agents. Preferred among these are isocyanate crosslinking agents.

The lower limit of the amount of the crosslinking agent relative to 100 parts by weight of the acrylic polymer is preferably 0.01 parts by weight, and the upper limit thereof is preferably 10 parts by weight. The lower limit is more preferably 0.1 parts by weight, and the upper limit is more preferably 5 parts by weight.

The adhesive composition of the present invention may contain a silane coupling agent to improve the adhesion strength. The silane coupling agent is not limited. Examples thereof include epoxy silanes, acrylic silanes, methacrylic silanes, aminosilanes, and isocyanate silanes.

The adhesive composition of the present invention may contain a colorant to impart light shielding properties. The colorant is not limited. Examples thereof include carbon black, aniline black, and titanium oxide. Preferred among these is carbon black, which is relatively inexpensive and chemically stable.

The adhesive composition of the present invention may optionally contain conventionally known fine particles and additives such as inorganic fine particles, electrically conductive fine particles, antioxidants, foaming agents, organic fillers, or inorganic fillers.

The present invention also encompasses an adhesive tape including an adhesive layer containing the adhesive composition of the present invention.

When the base polymer is the acrylic polymer, the gel fraction of the adhesive layer is not limited. The lower limit thereof is preferably 10% by weight, and the upper limit thereof is preferably 70% by weight. When the gel fraction is 10% by weight or greater, the adhesive layer has higher bulk strength and improved adhesion strength. When the gel fraction is 70% by weight or less, the adhesive layer can have improved interfacial wettability and can be less prone to interfacial peeling. The lower limit of the gel fraction is more preferably 15% by weight, and the upper limit thereof is more preferably 60% by weight. The lower limit is still more preferably 20% by weight, and the upper limit is still more preferably 50% by weight.

The gel fraction of the adhesive layer can be adjusted by, for example, adjusting the composition and the weight average molecular weight of the acrylic polymer and adjusting the type and amount of the crosslinking agent.

The gel fraction of the adhesive layer can be measured by the following method. The adhesive tape is cut to a flat rectangular shape with a size of 50 mm × 100 mm to prepare a specimen. The specimen is immersed in ethyl acetate at 23°C for 24 hours, then taken out of the ethyl acetate, and dried at 110°C for 1 hour. The weight of the dried specimen is measured, and the gel fraction is calculated using the following equation (1). No release film for protecting the adhesive layer is laminated on the specimen.

$$\text{Gel fraction (\% by weight)} = 100 \times (W_2 - W_0)/(W_1 - W_0) \quad (1)$$

($W_0$: weight of substrate, $W_1$: weight of specimen before immersion, $W_2$: weight of specimen after immersion and drying)

When the base polymer is the acrylic polymer, the lower limit of the shear storage modulus of the adhesive layer at 25°C measured using a dynamic viscoelastometer at a measurement frequency of 10 Hz (hereinafter also referred to simply as "shear storage modulus") is preferably $1.0 \times 10^4$ Pa, and the upper limit thereof is preferably $5.0 \times 10^5$ Pa.

When the shear storage modulus of the adhesive layer is within the above range, the adhesive layer has further

improved adhesion strength. The shear storage modulus of the adhesive layer is more preferably $3.0 \times 10^4$ Pa or greater, still more preferably $5.0 \times 10^4$ Pa or greater and is more preferably $4.0 \times 10^5$ Pa or less, still more preferably $3.5 \times 10^5$ Pa or less. The shear storage modulus of the adhesive layer can be adjusted by adjusting, for example, the type and polymerization ratio of the monomers that constitute the base polymer, the molecular weight of the base polymer, the gel fraction of the adhesive layer, the presence or absence of the tackifier resin (T2), and the type and amount of the compound (T1) of the present invention and the tackifier resin (T2).

The shear storage modulus of the adhesive layer can be measured by the following method.

First, a measurement sample consisting only of the adhesive layer is prepared. A dynamic viscoelastic spectrum from -50°C to 200°C of the obtained measurement sample is measured using a dynamic viscoelastometer such as viscoelastic spectrometer (e.g., DVA-200, produced by IT Measurement Co., Ltd. or its equivalent) at 5°C/min and a measurement frequency of 10 Hz in a low-heating-rate, shear deformation mode, and the storage modulus at 25°C is determined.

When the base polymer is the acrylic polymer, the loss tangent of the adhesive layer measured using a dynamic viscoelastometer at a measurement frequency of 10 Hz (tan$\delta$, hereinafter also referred to simply as "loss tangent") preferably has a peak at a temperature of -20°C or higher and 20°C or lower.

When the loss tangent of the adhesive layer has a peak in the above range, the adhesive layer can more easily have both adhesive force and holding power. The loss tangent more preferably has a peak at 15°C or lower, still more preferably 12°C or lower. The loss tangent more preferably has a peak at -15°C or higher, still more preferably -10°C or higher.

The loss tangent of the adhesive layer can be determined by measuring a dynamic viscoelastic spectrum from -100°C to 200°C using a viscoelastic spectrometer (e.g., DVA-200, produced by IT Measurement Co., Ltd. or its equivalent) at 5°C/min and a measurement frequency of 10 Hz in a low-heating-rate, shear deformation mode.

The thickness of the adhesive layer is not limited. The lower limit thereof is preferably 20 um, and the upper limit thereof is preferably 100 um. The lower limit is more preferably 25 um, and the upper limit is more preferably 80 um. When the thickness of the adhesive layer is within the range, the adhesive layer can have sufficient adhesion strength.

The thickness of the adhesive layer can be measured using a dial thickness gauge (e.g., "ABS Digimatic Indicator", produced by Mitutoyo Corporation).

The adhesive tape of the present invention may include a substrate. In this case, the adhesive layer may be laminated on one or both surfaces of the substrate.

The substrate is not limited. Examples thereof include resin films. The resin film is not limited. Examples thereof include polyolefin resin films such as polyethylene films and polypropylene films, polyester resin films such as polyethylene terephthalate (PET) films, ethylene-vinyl acetate copolymer films, polyvinyl chloride resin films, and polyurethane resin films. Examples of the substrate also include polyolefin foam sheets such as polyethylene foam sheets and polypropylene foam sheets and polyurethane foam sheets. Preferred among these are PET films.

The thickness of the substrate is not limited. The lower limit thereof is preferably 5 um, and the upper limit thereof is preferably 30 um. The lower limit is more preferably 8 um, and the upper limit is more preferably 20 $\mu$m.

The adhesive tape of the present invention may optionally include a different layer other than the adhesive layer and the substrate.

The method for producing the adhesive tape of the present invention is not limited. For example, the adhesive tape including the adhesive layer on both surfaces of the substrate may be produced by the following method.

First, a solvent is added to materials such as the base polymer, the compound (T1) of the present invention, the tackifier resin (T2), and the crosslinking agent to prepare a solution of an adhesive composition A. The solution of the adhesive composition A is applied to a surface of the substrate, and the solvent in the solution is completely removed by drying to form an adhesive layer A. Next, a release film is placed on the adhesive layer A such that the release-treated surface of the release film faces the adhesive layer A.

Subsequently, another release film is provided. A solution of an adhesive composition B is applied to the release-treated surface of the release film. The solvent in the solution is completely removed by drying. This produces a laminated film including an adhesive layer B on a surface of the release film. The obtained laminated film is placed on the rear surface of the substrate on which the adhesive layer A is formed, such that the adhesive layer B faces the rear surface of the substrate. Thus, a laminate is produced. The laminate is pressurized using a rubber roller or the like. This can produce a double-sided adhesive tape in which adhesive layers are on both surfaces of a substrate and release films cover the surfaces of the adhesive layers.

Alternatively, two laminated films may be produced in the same manner as above and placed on both surfaces of the substrate such that the adhesive layer of each laminated film faces the substrate. The resulting laminate may be pressurized using a rubber roller or the like. This can produce a double-sided adhesive tape in which adhesive layers are on both surfaces of a substrate and release films cover the surfaces of the adhesive layers.

The adhesive composition of the present invention and the adhesive tape of the present invention may be used in any applications. Having high adhesion strength, particularly, even to low polarity adherends (e.g., hard-to-bond

adherends such as polyolefin resin adherends and fluororesin adherend), the adhesive composition and the adhesive tape can be used to fix electronic device components or in-vehicle components. Specifically, for example, the adhesive composition and the adhesive tape can be used to fix components in television sets, monitors, portable electronic devices, and in-vehicle electronic devices.

The shape of the adhesive tape of the present invention in these applications is not limited. Examples thereof include square, rectangle, frame, circular, elliptical, and doughnut shapes.

- Advantageous Effects of Invention

The present invention can provide a compound capable of increasing the adhesion strength of adhesive compositions, particularly, even to low polarity adherends. The present invention can also provide a method for producing the compound, an adhesive composition containing the compound, and an adhesive tape including an adhesive layer containing the adhesive composition.

DESCRIPTION OF EMBODIMENTS

The embodiments of the present invention are more specifically described in the following with reference to examples. These examples are not intended to limit the present invention.

(Synthesis Example 1)

(Preparation of acrylic polymer)

A reactor equipped with a thermometer, a stirrer, and a condenser was charged with 100 parts by weight of ethyl acetate, purged with nitrogen, and then heated to start reflux. Thirty minutes after the ethyl acetate came to a boil, 0.08 parts by weight of azobisisobutyronitrile as a polymerization initiator was added. The monomer mixture shown in Table 1 was then uniformly and gradually dropped over 1.5 hours for reaction. Thirty minutes after the termination of dropping, 0.1 parts by weight of azobisisobutyronitrile was added to continue the polymerization reaction for an additional 5 hours. The contents of the reactor were then cooled while being diluted by adding ethyl acetate into the reactor, whereby an acrylic polymer solution having a solid content of 25% by weight was obtained.

The obtained acrylic polymer solution was filtered through a filter (material: polytetrafluoroethylene, pore size: 0.2 μm). The obtained filtrate was supplied to a gel permeation chromatograph (2690 Separations Model, produced by Waters) for GPC measurement at a sample flow rate of 1 mL/min and a column temperature of 40°C. The polystyrene equivalent molecular weight of the acrylic polymer was measured, and the weight average molecular weight (Mw) and the molecular weight distribution (Mw/Mn) were determined. A column used was GPC KF-806L (produced by Showa Denko K.K.). A detector used was a differential refractometer.

(Synthesis Example 2)

(Preparation of acrylic polymer)

An acrylic polymer was obtained as in Synthesis Example 1 except that the amount of ethyl acetate added was changed to 50 parts by weight.

(Synthesis Example 3)

(Preparation of acrylic polymer)

An acrylic polymer was obtained as in Synthesis Example 1 except that the monomer mixture was changed as shown in Table 1.

[Table 1]

| | | Synthesis Example 1 | Synthesis Example 2 | Synthesis Example 3 |
|---|---|---|---|---|
| Acrylic polymer [% by weight] | 2EHA (2-ethylhexyl acrylate) | 96.9 | 96.9 | 80.0 |
| | AAc (acrylic acid) | 3 | 3 | 1 |
| | HEA (2-hydroxyethyl acrylate) | 0,1 | 0,1 | 19 |
| Weight average molecular weight | Mw [$\times 10^4$] | 53 | 148 | 50 |
| Molecular weight distribution | Mw/Mn | 4.8 | 4.1 | 5.1 |

(Synthesis Example A)

(Preparation of compound (T1))

A reactor equipped with a thermometer, a stirrer, and a condenser was charged with 50 parts by weight of toluene, purged with nitrogen, and then heated to start reflux. After 30 minutes, while the toluene was kept at 75°C, 2 parts by weight of aluminum chloride ($AlCl_3$) was added. Then, a solution containing 22.3 parts by weight of the monomer (a) and 27.7 parts by weight of the monomer (b) (the molar ratio was as shown in Table 2) shown in Table 2 in 50 parts by weight of toluene was gradually dropped over 1.5 hours for reaction. After 4 hours of polymerization reaction, the contents of the reactor were cooled while 0.1 parts by weight of pyridine was added into the reactor to neutralize hydrochloric acid produced from aluminum chloride ($AlCl_3$). The precipitate resulting from neutralization was filtered out, and the obtained filtrate was subjected to liquid separation. Toluene was then evaporated, whereby a solid compound (T1) was obtained. For the monomer (a), its solvation free energy $\Delta\mu$ with polytetrafluoroethylene was calculated using molecular dynamics.

$^1$H-NMR measurement of the obtained compound (T1) showed that the compound (T1) was a copolymer including a structural unit (A) derived from pyrocatechol, the monomer (a), and a structural unit (B) derived from $\alpha$-pinene, the monomer (b).

A solution of the compound (T1) in tetrahydrofuran was filtered through a filter (material: polytetrafluoroethylene, pore size: 0.2 um). The obtained filtrate was supplied to a gel permeation chromatograph (2690 Separations Model, produced by Waters) for GPC measurement at a sample flow rate of 1 mL/min and a column temperature of 40°C. The polystyrene equivalent molecular weight of the compound (T1) was measured, and the weight average molecular weight (Mw) was determined. A column used was GPC KF-802.5L (produced by Showa Denko K.K.). A detector used was a differential refractometer.

The obtained compound (T1) was subjected to measurement using a differential scanning calorimeter (SII Exstar 6000/DSC 6220, produced by Hitachi High-Tech Science Corporation) in a nitrogen atmosphere at a heating rate of 10°C/min. The value obtained in the first run was used to determine the glass transition temperature.

A mold having a size of 10 $\times$ 50 mm was filled with the obtained compound (T1) and melted at a temperature 100°C higher than the glass transition temperature of the compound to form a specimen having a thickness of 1 mm. This specimen was subjected to a tensile test using a tensile tester (TENSILON, produced by ORIENTEC) at a tensile speed of 200 mm/min, a clamp distance of 15 mm, and 25°C, whereby the Young's modulus at 25°C was measured.

First, 0.250 g of the obtained compound (T1) was weighed and diluted with 50 mL of cyclohexane. Next, 10.0 mL of a Wijs reagent (produced by Wako Pure Chemical Industries, Ltd., 0.1 mol/L iodine chloride/acetic acid solution) was added and sufficiently shaken. The mixture was left to stand for 30 minutes to allow reaction to proceed. Then, 10 mL of a 15% by weight aqueous potassium iodide solution and 30 mL of water were added and stirred. Further, a 0.1 N aqueous sodium thiosulfate solution (produced by Wako Pure Chemical Industries, Ltd.) was gradually dropped. When the solution turned pale yellow, three drops of a starch solution (10 g/L) was added. Thereafter, a 0.1 N aqueous sodium thiosulfate solution (produced by Wako Pure Chemical Industries, Ltd.) was gradually dropped (drop amount Y mL) until the solution was no longer blue. Subsequently, the drop amount (drop amount Z mL) for a blank was determined in the same manner except that no sample (compound (T1)) was added. The iodine value of the compound (T1) was determined using the following equation.

$$\text{Iodine value (g/100 g)} = (Z - Y) \times 1.269/0.250$$

The bio-derived carbon content of the obtained compound (T1) was measured in conformity with ASTM D6866-20.

(Synthesis Examples B to M and 0)

(Preparation of compound (T1))

A compound (T1) was obtained as in Synthesis Example A except that the monomers (a) and (b) were changed as shown in Table 2.

(Synthesis Example N)

(Preparation of compound (T1))

A reactor equipped with a thermometer, a stirrer, and a condenser was charged with 50 parts by weight of toluene, purged with nitrogen, and then heated to start reflux. After 30 minutes, while the toluene was kept at 75°C, 2 parts by weight of aluminum chloride ($AlCl_3$) was added. Then, a solution containing 50 parts by weight of the monomers (a) and (b) (the molar ratio was as shown in Table 2) shown in Table 2 in 50 parts by weight of toluene was gradually dropped over 1.5 hours for reaction. After 4 hours of polymerization reaction, the contents of the reactor were cooled while 0.1 parts by weight of pyridine was added into the reactor to neutralize hydrochloric acid produced from aluminum chloride ($AlCl_3$). The precipitate resulting from neutralization was filtered out, and the obtained filtrate was subjected to liquid separation. Toluene was then evaporated, whereby a solid compound (T1) was obtained.

$^1$H-NMR measurement of the obtained compound (T1) showed that the compound (T1) was a copolymer including a structural unit (A-2') derived from 4-vinylbenzoic acid, the monomer (a), and a structural unit (B) derived from α-pinene, the monomer (b).

[Table 2]

| | | | | Synthesis Example A | Synthesis Example B | Synthesis Example C | Synthesis Example D | Synthesis Example E | Synthesis Example F | Synthesis Example G | Synthesis Example H | Synthesis Example I | Synthesis Example J | Synthesis Example K | Synthesis Example L | Synthesis Example M | Synthesis Example N | Synthesis Example O |
|---|---|---|---|---|---|---|---|---|---|---|---|---|---|---|---|---|---|---|
| Compound (T1) [mol %] | Monomer (a) | Pyrocat echol | | 50 | - | - | - | - | - | - | - | - | - | - | - | 10 | - | - |
| | | Pyrogallol | | - | 60 | 50 | 30 | 10 | 3 | 0.5 | 10 | 10 | 10 | 10 | 10 | - | - | - |
| | | 4-Vinylbenzoic acid | | - | - | - | - | - | - | - | - | - | - | - | - | - | 10 | - |
| | | 1, 2, 3- Tri methoxybenzene | | - | - | - | - | - | - | - | - | - | - | - | - | - | - | 10 |
| | Monomer (b) | Terpene monomer | α - PInene | 50 | 40 | 50 | 70 | 90 | 97 | 99. 5 | - | - | 80 | 80 | - | - | - | - |
| | | | β - PInene | - | - | - | - | - | - | - | 90 | - | - | - | - | - | 90 | 90 |
| | | | Li nonene | - | - | - | - | - | - | - | - | 90 | - | - | - | - | - | - |
| | | Vinyl monomer | Styrene | - | - | - | - | - | - | - | - | - | 10 | - | 90 | - | - | - |
| | | Conjugated di ene monomer | Isoprene | - | - | - | - | - | - | - | - | - | - | 10 | - | 90 | - | - |
| Solvation free energy Δ μ of monomer (a) | kcal / mol | | | - 36 | - 58 | - 58 | -58 | - 58 | - 58 | - 58 | - 58 | -58 | - 58 | - 58 | -58 | -36 | -71 | -43 |
| Iodine value (C=C bond content) | g/ 100 g | | | 93 | 75 | 93 | 130 | 168 | 180 | 185 | 168 | 168 | 149 | 186 | 0 | 213 | 168 | 168 |

(continued)

| | | Synthesis Example A | Synthesi s Example B | Synthesi s Example C | Synthesi s Example D | Synthesi s Example E | Synthesi s Example F | Synthesi s Example G | Synthesi s Example H | Synthesi s Example I | Synthesi s Example J | Synthesi s Example K | Synthesi s Example L | Synthesi s Example M | Synthesi s Example N | Synthesi s Example O |
|---|---|---|---|---|---|---|---|---|---|---|---|---|---|---|---|---|
| Weight average moleculer weight | Mw | 790 | 510 | 540 | 530 | 560 | 620 | 710 | 1500 | 900 | 900 | 850 | 3500 | 1000 | 3800 | 1520 |
| Gass transition temperature | [°C] | 30 | 32 | 31 | 30 | 32 | 34 | 36 | 40 | 42 | 45 | 30 | 48 | -20 | 45 | 31 |
| Young's modulus (25° C) | [MPa] | 78 | 81 | 80 | 80 | 85 | 85 | 86 | 91 | 90 | 96 | 74 | 104 | 0.6 | 91 | 90 |
| Bio-derived carbon content | [%] | 63 | 100 | 100 | 100 | 100 | 100 | 100 | 100 | 100 | 91 | 95 | 8 | 0 | 92 | 97 |

(Example 1)

(1) Production of adhesive tape

Thirty parts by weight of the compound (T1) (Synthesis Example A) was added to 100 parts by weight of the solids of the acrylic polymer (Synthesis Example 1). Further, 30 parts by weight of ethyl acetate (produced by Fuji Chemicals Ltd.) and 2.5 parts by weight of an isocyanate crosslinking agent (produced by Nippon Polyurethane Industry Co., Ltd., product name "Coronate L45") were added and stirred to prepare an adhesive composition solution.

A release film having a thickness of 150 um was provided. The adhesive composition solution was applied to the release-treated side of the release film and dried at 100°C for five minutes to form an adhesive layer having a thickness of 50 um. This adhesive layer was bonded to a surface of a corona-treated PET film having a thickness of 50 um as a substrate. Subsequently, the same adhesive layer as above was bonded to the opposite surface of the substrate in the same manner. The adhesive layers were aged by heating at 40°C for 48 hours. This produced an adhesive tape in which the adhesive layers were laminated on both surfaces of the substrate and the release films covered the surfaces of the adhesive layers.

(2) Measurement of gel fraction

The adhesive tape was cut to a flat rectangular shape with a size of 50 mm × 100 mm to prepare a specimen. The specimen was immersed in ethyl acetate at 23°C for 24 hours, then taken out of the ethyl acetate, and dried at 110°C for 1 hour. The weight of the dried specimen was measured, and the gel fraction was calculated using the following equation (1). No release film for protecting the adhesive layers was laminated on the specimen.

$$\text{Gel fraction (\% by weight)} = 100 \times (W_2 - W_0)/(W_1 - W_0) \quad (1)$$

($W_0$: weight of substrate, $W_1$: weight of specimen before immersion, $W_2$: weight of specimen after immersion and drying)

(3) Measurement of shear storage modulus

A measurement sample consisting only of the adhesive layer was prepared. A dynamic viscoelastic spectrum from - 50°C to 200°C of the obtained measurement sample was measured using a viscoelastic spectrometer (DVA-200, produced by IT Measurement Co., Ltd.) at 5°C/min and a measurement frequency of 10 Hz in a low-heating-rate, shear deformation mode, and the storage modulus at 25°C was determined.

(4) Measurement of loss tangent (tanδ) peak temperature

A measurement sample consisting only of the adhesive layer was prepared. A dynamic viscoelastic spectrum from - 100°C to 200°C of the obtained measurement sample was measured using a viscoelastic spectrometer (DVA-200 produced by IT Measurement Co., Ltd.) at 5°C/min and a measurement frequency of 10 Hz in a low-heating-rate, shear deformation mode. From the obtained dynamic viscoelastic spectrum, the loss tangent (tanδ) peak temperature was determined.

(Examples 2 to 25 and Comparative Examples 1 and 2)

An adhesive tape was obtained as in Example 1 except that the type and amount of the acrylic polymer, the compound (T1), the tackifier resin (T2), and the crosslinking agent were changed as shown in Table 3. The tackifier resins (T2) and crosslinking agents used are as follows. For each tackifier resin (T2), the value of the smallest of the solvation free energies Δμ of the monomers constituting the tackifier resin (T2) with polytetrafluoroethylene is shown.

Rosin ester resin (produced by Arakawa Chemical Industries Ltd., product name "Pinecrystal KE359", Δμ = -5 kcal/mol)
Terpene phenolic resin (produced by Yasuhara Chemical Co., Ltd., product name "YS Polyster G150", Δμ = -25 kcal/mol)
Isocyanate crosslinking agent (produced by Nippon Polyurethane Industry Co., Ltd., product name "Coronate L45")
Epoxy crosslinking agent (produced by Mitsubishi Gas Chemical Company, Inc., product name "Tetrad E5XM")

(Example 26)

Thirty parts by weight of the compound (T1) (Synthesis Example A) was added to 100 parts by weight of the solids of a styrene elastomer (SIS block copolymer, produced by Zeon Corporation, Quintac 3520, styrene content: 15% by weight, diblock proportion: 78% by weight). Further, 30 parts by weight of toluene (produced by Fuji Chemicals Ltd.) was added and stirred to prepare an adhesive composition solution.

A release film having a thickness of 150 um was provided. The adhesive composition solution was applied to the release-treated side of the release film and dried at 100°C for five minutes to form an adhesive layer having a thickness of 50 um. This adhesive layer was bonded to a surface of a corona-treated PET film having a thickness of 50 um as a substrate. Subsequently, the same adhesive layer as above was bonded to the opposite surface of the substrate in the same manner. The adhesive layers were aged by heating at 40°C for 48 hours. This produced an adhesive tape in which the adhesive layers were laminated on both surface of the substrate and the release films covered the surfaces of the adhesive layers.

(Examples 27 to 57 and Comparative Examples 3 and 4)

An adhesive tape was obtained as in Example 26 except that the type and amount of the styrene elastomer, the compound (T1), and the tackifier resin (T2) were changed as shown in Table 4 or 5. The styrene elastomers and tackifier resin (T2) used are as follows. For the tackifier resin (T2), the value of the smallest of the solvation free energies $\Delta\mu$ of the monomers constituting the tackifier resin (T2) with polytetrafluoroethylene is shown.

Styrene elastomer (SIS block copolymer, produced by Zeon Corporation, Quintac 3520, styrene content: 15% by weight, diblock proportion: 78% by weight)
Styrene elastomer (SIS block copolymer, produced by Zeon Corporation, Quintac 3433N, styrene content: 16% by weight, diblock proportion: 56% by weight)
Styrene elastomer (SIS block copolymer, produced by Zeon Corporation, Quintac 3421, styrene content: 14% by weight, diblock proportion: 26% by weight)
Styrene elastomer (SIS block copolymer, produced by Zeon Corporation, Quintac 3450, styrene content: 19% by weight, diblock proportion: 30% by weight)
Styrene elastomer (SIS block copolymer, produced by Zeon Corporation, Quintac 3280, styrene content: 25% by weight, diblock proportion: 17% by weight)
Styrene elastomer (SBS block copolymer, produced by Kraton Performance Polymer Japan, Kraton DX410, styrene content: 18% by weight, diblock proportion: 60% by weight)
Terpene resin (produced by Yasuhara Chemical Co., Ltd., product name "YS Resin PX 1150", $\Delta\mu$ = -5 kcal/mol)

<Evaluation>

The adhesive tapes obtained in the examples and the comparative examples were evaluated as follows. Tables 3 to 5 show the results.

(1) 180° Peel test

The adhesive tape was cut into a 25-mm-wide specimen. The adhesive layer of the obtained specimen was placed on a stainless steel (SUS304) plate (produced by Nippon Testpanel Co., Ltd.), a polypropylene (PP) plate (produced by Nippon Testpanel Co., Ltd.), or a polytetrafluoroethylene (PTFE) plate (produced by Nippon Testpanel Co., Ltd.). Subsequently, a 2-kg rubber roller was moved back and forth once on the specimen at a rate of 300 mm/min to bond the specimen and the stainless steel (SUS304) plate, polypropylene (PP) plate, or polytetrafluoroethylene (PTFE) plate. The specimen was then left to stand at 23°C for one hour to prepare a test sample. The test sample after standing was subjected to a tensile test in the 180° direction at a peeling rate of 300 mm/min in conformity with JIS Z0237, and the peeling force was measured.

180° Peel test for SUS

∞ (Excellent): peeling force of 20 N/inch or greater
o (Good): peeling force of 15 N/inch or greater and less than 20 N/inch
Δ (Fair): peeling force of 10 N/inch or greater and less than 15 N/inch

× (Poor): peeling force of less than 10 N/inch

180° Peel test for PP

∞ (Excellent): peeling force of 15 N/inch or greater
o (Good): peeling force of 10 N/inch or greater and less than 15 N/inch
Δ (Fair): peeling force of 5 N/inch or greater and less than 10 N/inch
× (Poor): peeling force of less than 5 N/inch

180° Peel test for PTFE

∞ (Excellent): peeling force of 5 N/inch or greater
o (Good): peeling force of 3 N/inch or greater and less than 5 N/inch
Δ (Fair): peeling force of 1 N/inch or greater and less than 3 N/inch
× (Poor): peeling force of less than 1 N/inch

(2) Alkali resistance test

The adhesive tape was cut into a size of 25 mm × 75 mm, and the release film on one surface was removed. The tape was bonded, as a backing, to a polyethylene terephthalate (PET) film having a thickness of 23 um to prepare a specimen. In an environment at 23°C, the release film covering the other adhesive surface of the specimen was removed, and the specimen was compression bonded to a surface of a stainless-steel (SUS304) plate by moving a 2-kg roller back and forth once thereon. Thus, a test sample before chemical solution immersion was obtained. Sodium hydroxide was diluted with ion-exchanged water to prepare an alkaline chemical solution having a pH of 12. The test sample before chemical solution immersion was immersed in the alkaline chemical solution for one day in an atmosphere of 60°C. The test sample was then taken out from the alkaline chemical solution, washed with ion-exchanged water, and then dried at 23°C for one hour to prepare a test sample after chemical solution immersion. The obtained test sample before and after chemical solution immersion was checked for peeling of the adhesive tape from the stainless-steel plate.

◦ (Good): The adhesive tape did not peel off at all.
Δ (Fair): The adhesive tape only slightly peeled off at its edge.
× (Poor): The entire adhesive tape peeled off.

[Table 3]

| | | Example | | | | | | | | | | | | | | | | | | | | | | | | | Comparative Example | |
|---|---|---|---|---|---|---|---|---|---|---|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 1 | 2 |
| Base polymer [% by weight] | Synthesis Example 1 (acrylic polymer) | 100 | 100 | 100 | 100 | 100 | 100 | 100 | 100 | 100 | 100 | 100 | 100 | 100 | 100 | 100 | 100 | - | - | 100 | 100 | 100 | 100 | 100 | 100 | 100 | 100 | 100 |
| | Synthesis Example 2 (acrylic polymer) | - | - | - | - | - | - | - | - | - | - | - | - | - | - | - | - | 100 | - | - | - | - | - | - | - | - | - | - |
| | Synthesis Example 3 (acrylic polymer) | - | - | - | - | - | - | - | - | - | - | - | - | - | - | - | - | - | 100 | - | - | - | - | - | - | - | - | - |
| Compound (T1) [% by weight] | Synthesis Example A (pyrocatechol/α-pinene) | 30 | - | - | - | - | - | - | - | - | - | - | - | - | - | - | - | - | - | - | - | - | - | - | - | - | - | - |
| | Synthesis Example B (pyrogallol 60 mol%/α-pinene) | - | 30 | - | - | - | - | - | - | - | - | - | - | - | - | - | - | - | - | - | - | - | - | - | - | - | - | - |
| | Synthesis Example C (pyrogallol 50 mol%/α-pinene) | - | - | 30 | - | - | - | - | - | - | - | - | 1 | 10 | 35 | 10 | 40 | 30 | 30 | 30 | 30 | 30 | - | - | - | - | - | - |
| | Synthesis Example D (pyrogallol 30 mol%/α-pinene) | - | - | - | 30 | - | - | - | - | - | - | - | - | - | - | - | - | - | - | - | - | - | - | - | - | - | - | - |
| | Synthesis Example E (pyrogallol 10 mol%/α-pinene) | - | - | - | - | 30 | - | - | - | - | - | - | - | - | - | - | - | - | - | - | - | - | - | - | - | - | - | - |
| | Synthesis Example F (pyrogallol 3 mol%/α-pinene) | - | - | - | - | - | 30 | - | - | - | - | - | - | - | - | - | - | - | - | - | - | - | - | - | - | - | - | - |
| | Synthesis Example G (pyrogallol 0.5 mol%/α-pinene) | - | - | - | - | - | - | 30 | - | - | - | - | - | - | - | - | - | - | - | - | - | - | - | - | - | - | - | - |
| | Synthesis Example H (pyrogallol/β-pinene) | - | - | - | - | - | - | - | 30 | - | - | - | - | - | - | - | - | - | - | - | - | - | - | - | - | - | - | - |
| | Synthesis Example I (pyrogallol/limonene) | - | - | - | - | - | - | - | - | 30 | - | - | - | - | - | - | - | - | - | - | - | - | - | - | - | - | - | - |
| | Synthesis Example J (pyrogallol/α-pinene/styrene) | - | - | - | - | - | - | - | - | - | 30 | - | - | - | - | - | - | - | - | - | - | - | - | - | - | - | - | - |
| | Synthesis Example K (pyrogallol/α-pinene/isoprene) | - | - | - | - | - | - | - | - | - | - | 30 | - | - | - | - | - | - | - | - | - | - | - | - | - | - | - | - |
| | Synthesis Example L (pyrogallol/styrene) | - | - | - | - | - | - | - | - | - | - | - | - | - | - | - | - | - | - | - | - | - | 30 | - | - | - | - | - |
| | Synthesis Example M (pyrocatechol/isoprene) | - | - | - | - | - | - | - | - | - | - | - | - | - | - | - | - | - | - | - | - | - | - | 30 | - | - | - | - |
| | Synthesis Example N (vinylbenzoic acid/β-pinene) | - | - | - | - | - | - | - | - | - | - | - | - | - | - | - | - | - | - | - | - | - | - | - | 30 | - | - | - |
| | Synthesis Example O (trimethoxybenzene/β-pinene) | - | - | - | - | - | - | - | - | - | - | - | - | - | - | - | - | - | - | - | - | - | - | - | - | 30 | - | - |
| Tackifier resin (T2) [% by weight] | KE359 (rosin ester resin) | - | - | - | - | - | - | - | - | - | - | - | - | - | - | - | - | - | - | - | - | - | - | - | - | - | 30 | - |
| | G150 (terpene phenolic resin) | - | - | - | - | - | - | - | - | - | - | - | 39 | 30 | 5 | 50 | - | - | - | - | - | - | - | - | - | - | - | 30 |
| Crosslinking agent [% by weight] | L-45 (isocyanate crosslinking agent) | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 1.0 | 0.5 | 4.0 | - | 2.5 | 2.5 | 2.5 | 2.5 | 2.5 | 2.5 |
| | E5XM (epoxy crosslinking agent) | - | - | - | - | - | - | - | - | - | - | - | - | - | - | - | - | - | - | - | - | 0.5 | - | - | - | - | - | - |
| Physical properties | Shear storage modulus (25° C) [Pa/$10^5$] | 0.7 | 1.1 | 0.9 | 0.9 | 0.8 | 0.8 | 0.8 | 1.0 | 0.9 | 1.0 | 0.8 | 4.1 | 3.5 | 1.7 | 4.8 | 1.0 | 0.9 | 1.0 | 0.8 | 1.0 | 1.2 | 1.1 | 0.7 | 1.2 | 0.9 | 2.0 | 3.1 |
| | Loss tangent peak temperature [° C] | -9 | 7 | 3 | -1 | -5 | -6 | -6 | 0 | -3 | 4 | -7 | 22 | 19 | 8 | 29 | 7 | 4 | 13 | 3 | 3 | 3.0 | 8 | -15 | 5 | -10 | 0 | 17 |
| | Gel fraction [% by weight] | 30 | 20 | 25 | 30 | 35 | 35 | 40 | 25 | 25 | 25 | 25 | 35 | 30 | 20 | 30 | 20 | 25 | 25 | 10 | 70 | 80 | 35 | 30 | 35 | 40 | 30 | 30 |
| Evaluation | 180° Peel test for SUS | ○ | ○ | ○○ | ○○ | ○○ | ○ | ○ | ○○ | ○○ | ○○ | ○○ | ○○ | ○○ | ○○ | ○ | ○ | ○○ | ○○ | ○○ | ○○ | ○ | ○ | ○ | ○○ | ○○ | ○ | ○ |
| | 180° Peel test for PP | △ | ○ | ○○ | ○○ | ○○ | ○ | △ | ○○ | ○○ | ○○ | ○○ | ○ | ○○ | ○○ | ○ | ○ | ○○ | ○○ | ○○ | ○○ | ○ | △ | △ | ○○ | ○○ | × | × |
| | 180° Peel test for PTFE | △ | ○ | ○○ | ○○ | ○○ | ○ | △ | ○○ | ○○ | ○○ | ○○ | △ | ○○ | ○○ | △ | ○ | ○○ | ○○ | ○○ | ○○ | ○ | △ | △ | ○○ | ○ | × | × |

[Table 4a]

| | | Example | | | | | | | | | |
|---|---|---|---|---|---|---|---|---|---|---|---|
| | | 26 | 27 | 28 | 29 | 30 | 31 | 32 | 33 | 34 | 35 |
| Base polymer [% by weight] | SIS: Quintac 3520 Styrene content: 15% by weight Diblock proportion: 78% by weight | 100 | 100 | 100 | 100 | 100 | 100 | 100 | 100 | 100 | 100 |
| | SIS: Quintac 3433N Styrene content: 16% by weight Diblock proportion: 56% by weight | - | - | - | - | - | - | - | - | - | - |
| | SIS: Quintac 3421 Styrene content: 14% by weight Diblock proportion: 26% by weight | - | - | - | - | - | - | - | - | - | - |
| | SIS: Quintac 3450 Styrene content: 19% by weight Diblock proportion: 30% by weight | - | - | - | - | - | - | - | - | - | - |
| | SIS: Quintac 3280 Styrene content: 25% by weight Diblock proportion: 17% by weight | - | - | - | - | - | - | - | - | - | - |
| | SBS: Kraton DX410 Styrene content: 18% by weight Diblock proportion: 60% by weight | - | - | - | - | - | - | - | - | - | - |

(continued)

| | | Example | | | | | | | | | |
|---|---|---|---|---|---|---|---|---|---|---|---|
| | | 26 | 27 | 28 | 29 | 30 | 31 | 32 | 33 | 34 | 35 |
| Compound (T1) [% by weight] | Synthesis Example A (pyrocatechol/a-pinene) | 30 | - | - | - | - | - | - | - | - | - |
| | Synthesis Example B (pyrogallol 60 mol%/α-pinene) | - | 30 | | | | - | | | | |
| | Synthesis Example C (pyrogallol 50 mol%/α-pinene) | - | - | 30 | - | - | - | - | - | - | - |
| | Synthesis Example D (pyrogallol 30 mol%/α-pinene) | - | - | - | 30 | | | - | | | - |
| | Synthesis Example E (pyrogallol 10 mol%/α-pinene) | - | - | - | - | 30 | - | - | - | - | - |
| | Synthesis Example F (pyrogallol 3 mol%/α-pinene) | - | - | - | - | - | 30 | - | - | - | - |
| | Synthesis Example G (pyrogallol 0.5 mol%/α-pinene) | - | - | - | - | - | - | 30 | - | - | - |
| | Synthesis Example H (pyrogallol/β-pinene) | - | - | - | - | - | - | - | 30 | - | - |
| | Synthesis Example I (pyrogallol/limonene) | - | - | - | - | - | - | - | - | 30 | |
| | Synthesis Example J (pyrogallol/α-pinene/ styrene) | - | - | - | - | - | - | - | - | - | 30 |
| | Synthesis Example K (pyrogallol/α-pinene/ isoprene) | - | - | - | - | - | - | - | - | - | - |
| | Synthesis Example L (pyrogallol/styrene) | - | - | - | - | - | - | - | - | - | - |
| | Synthesis Example M (pyrocatechol/ isoprene) | - | - | - | | - | - | - | - | - | - |
| | Synthesis Example N (vinylbenzoic acid/β-pinene) | - | - | - | - | - | - | - | - | - | - |
| | Synthesis Example O (trimethoxybenzene/β-pinene) | - | - | - | - | - | - | - | - | - | - |

(continued)

| | | Example | | | | | | | | | |
|---|---|---|---|---|---|---|---|---|---|---|---|
| | | 26 | 27 | 28 | 29 | 30 | 31 | 32 | 33 | 34 | 35 |
| Tackifier resin (T2) [% by weight] | PX1150(terpene resin) | - | - | - | - | - | - | - | - | - | - |
| | KE359 (rosi n ester resin) | - | - | - | - | - | - | - | - | - | - |
| | G150 (terpene phenolic resin) | - | - | - | - | - | - | - | - | - | - |
| Physical properties | Shear storage modulus (25°C) [Pa/$10^5$] | 1.9 | 1.8 | 1.9 | 1.8 | 1.7 | 1.7 | 1.7 | 1.8 | 1.8 | 1.7 |
| | Loss tangent peak temperature [°C] | -34 | -31 | -31 | -32 | -31 | -32 | -33 | -31 | -31 | -29 |
| Evaluation | 180° Peel test for SUS | ○ | ○ | ○○ | ○○ | ○○ | ○ | ○ | ○○ | ○○ | ○○ |
| | 180° Peel test for PP | Δ | ○○ | ○○ | ○○ | ○○ | ○ | Δ | ○○ | ○○ | ○○ |
| | 180° Peel test for PTFE | Δ | Δ | ○ | ○ | ○ | Δ | Δ | ○ | ○ | ○ |
| | Alkali resistance test | Δ | ○ | ○ | ○ | ○ | ○ | Δ | ○ | ○ | ○ |

[Table 4b]

| | | Examples | | | | | | | | | | | |
|---|---|---|---|---|---|---|---|---|---|---|---|---|---|
| | | 36 | 37 | 38 | 39 | 40 | 41 | 42 | 43 | 44 | 45 | 46 | 47 |
| Base polymer [% by weight] | SIS: Quintac 3520<br>Styrene content: 15% by weight<br>Diblock proportion: 78% by weight | 100 | 100 | 100 | 100 | 100 | 100 | 100 | 100 | 100 | 100 | - | - |
| | SIS: Quintac 3433N<br>Styrene content: 16% by weight<br>Diblock proportion: 56% by weight | - | - | - | - | - | - | - | - | - | - | 100 | - |
| | SIS: Quintac 3421<br>Styrene content: 14% by weight<br>Diblock proportion: 26% by weight | - | - | - | - | - | - | - | - | - | - | - | 100 |
| | SIS: Quintac 3450<br>Styrene content: 19% by weight<br>Diblock proportion: 30% by weight | - | - | - | - | - | - | - | - | - | - | - | - |
| | SIS: Quintac 3280<br>Styrene content: 25% by weight<br>Diblock proportion: 17% by weight | - | - | - | - | - | - | - | - | - | - | - | - |
| | SBS: Kraton DX410<br>Styrene content: 18% by weight<br>Diblock proportion: 60% by weight | - | - | - | - | - | - | - | - | - | - | - | - |
| | Synthesis Example A (pyrocatechol/α-pinene) | - | - | - | - | - | - | - | - | - | - | - | - |
| | Synthesis Example B (pyrogallol 60 mol%/α-pinene) | - | - | - | - | - | - | - | - | - | - | - | - |
| | Synthesis Example C (pyrogallol 50 mol%/α-pinene) | 1 | 10 | 35 | 40 | 10 | 10 | 10 | 10 | 10 | 10 | 30 | 30 |
| | Synthesis Example D (pyrogallol 30 mol%/α-pinene) | | - | - | - | - | - | - | - | - | - | - | - |
| | Synthesis Example E (pyrogallol 10 mol%/α-pinene) | - | - | - | - | - | - | - | - | - | - | - | - |

(continued)

| | | Examples | | | | | | | | | | | |
|---|---|---|---|---|---|---|---|---|---|---|---|---|---|
| | | 36 | 37 | 38 | 39 | 40 | 41 | 42 | 43 | 44 | 45 | 46 | 47 |
| Compound (T1) [% by weight] Tackifier resin (T2) [% by weight! | Synthesis Example F (pyrogallol 3 mol%/α-pinene) | - | - | - | - | - | - | - | - | - | - | - | - |
| | Synthesis Example G (pyrogallol 0.5 mol%/α-pinene) | - | - | - | - | - | - | - | - | - | - | - | - |
| | Synthesis Example H (pyroqallol/β-pinene) | - | - | - | - | - | - | - | - | - | - | - | - |
| | Synthesis Example I (pyrogallol/ limonene) | - | - | - | - | - | - | - | - | - | - | - | - |
| | Synthesis Example J (pyrogallol/α-pinene/styrene) | - | - | - | - | - | - | - | - | - | - | - | - |
| | Synthesis Example K (pyrogallol/α-pinene/isoprene) | - | - | - - | - | - | - | - | - | - | - | - | - |
| | Synthesis Example L (pyrogallol/ styrene) | - | - | - | - | - | - | - | - | - | - | - | - |
| | Synthesis Example M (pyrocatechol/ isoprene) | - | - | - | - | - | - | - | - | - | - | - | - |
| | Synthesis Example N (vinylbenzoic acid/β-pinene) | - | - | - | - | - | - | - | - | - | - | - | - |
| | Synthesis Example O (trimethoxybenzene/p-pinene) | - | - | - | - | - | - | - | - | - | - | - | - |
| | PX1150 (terpene resin) | 39 | 30 | 5 | - | - | 40 | 90 | 100 | - | - | - | . |
| | KE359 (rosin ester resin) | - | - | - | - | - | - | - | - | 30 | - | - | - |
| | G150 (terpene phenolic resin) | - | - | - | - | - | - | - | - | - | 30 | - | - |
| Physical properties | Shear storage modulus (25°C) [Pa/$10^5$] | 2.3 | 2.0 | 1.9 | 2.5 | 3.7 | 2.3 | 4.0 | 4.6 | 2.1 | 2.5 | 1.8 | 1.8 |
| | Loss tangent peak temperature [°C] | -17 | -19 | -27 | -28 | -40 | -13 | 17 | 21 | -21 | -8 | -30 | -28 |

(continued)

| | | Examples | | | | | | | | | | | |
|---|---|---|---|---|---|---|---|---|---|---|---|---|---|
| | | 36 | 37 | 38 | 39 | 40 | 41 | 42 | 43 | 44 | 45 | 46 | 47 |
| Evaluation | 180° Peel test for SUS | ○○ | ○○ | ○○ | ○○ | ○ | ○○ | ○○ | ○ | ○○ | ○○ | ○○ | ○ |
| | 180° Peel test for PP | ○ | ○○ | ○○ | ○ | ○ | ○○ | ○○ | ○ | ○○ | ○○ | ○○ | Δ |
| | 180° Peel test for PTFE | ○ | ○○ | ○ | Δ | Δ | ○○ | ○○ | Δ | ○○ | ○○ | ○ | Δ |
| | Alkali resistance test | Δ | ○ | ○ | ○ | ○ | ○ | ○ | ○ | ○ | ○ | ○ | ○ |

[Table 5]

| | | Example | | | | | | | | | | Comparative Example | |
|---|---|---|---|---|---|---|---|---|---|---|---|---|---|
| | | 48 | 49 | 50 | 51 | 52 | 53 | 54 | 55 | 56 | 57 | 3 | 4 |
| Base polymer [% by weight] | SIS: Quintac 3520<br>Styrene content: 15% by weight<br>Diblock proportion: 78% by weight | - | - | - | - | 100 | 100 | 100 | 100 | - | - | 100 | 100 |
| | SIS: Quintac 3433N<br>Styrene content: 16% by weight<br>Diblock proportion: 56% bv weight | - | - | - | - | - | - | - | - | - | - | - | - |
| | SIS: Quintac 3421<br>Styrene content: 14% by weight<br>Diblock proportion: 26% bv weight | - | - | - | - | - | - | - | - | - | - | - | - |
| | SIS: Quintac 3450<br>Styrene content: 19% by weight<br>Diblock proportion: 30% bv weight | 100 | 100 | 100 | 100 | - | - | - | - | - | - | - | - |
| | SIS: Quintac 3280<br>Styrene content: 25% by weight<br>Diblock proportion: 17% by weight | - | - | - | - | - | - | - | - | 100 | - | - | - |
| | SBS: Kraton DX410<br>Styrene content: 18% by weight<br>Diblock proportion: 60% by weight | - | - | - | - | - | - | - | - | - | 100 | - | - |
| Compound (T1) [% by weight] | Synthesis Example A (pyrocatechol/α-pinene) | - | - | - | - | - | - | - | - | - | - | - | - |
| | Synthesis Example B (pyroctallol 60 mol%/α-pinene) | - | - | - | - | - | - | - | - | - | - | - | - |
| | Synthesis Example C (pyrogallol 50 mol%/α-pinene) | 10 | 10 | 10 | 10 | - | - | - | - | 30 | 30 | - | - |
| | Synthesis Example D (pyrogallol 30 mol%/α-pinene) | - | - | - | - | - | - | - | - | - | - | - | - |
| | Synthesis Example E (pyrogallol 10 mol%/α-pinene) | - | - | - | - | - | - | - | - | - | - | - | - |
| | Synthesis Example F (pyrogallol 3 mol%/α-pinene) | - | - | - | - | - | - | - | - | - | - | - | - |
| | Synthesis Example G (pyrogallol 0.5 mol%/α-pinene) | - | - | - | - | - | - | - | - | - | - | - | - |
| | Synthesis Example H (pyrogallol/β-pinene) | - | - | - | - | - | - | - | - | - | - | - | - |

(continued)

| | | Example | | | | | | | | | | Comparative Example | |
|---|---|---|---|---|---|---|---|---|---|---|---|---|---|
| | | 48 | 49 | 50 | 51 | 52 | 53 | 54 | 55 | 56 | 57 | 3 | 4 |
| | Synthesis Example I (pyrogallol/limonene) | - | - | - | - | - | - | - | - | - | - | - | - |
| | Synthesis Example J (pyrogallol/α-pinene/styrene) | - | - | - | - | - | - | - | - | - | - | - | - |
| | Synthesis Example K (pyrogallol/α-pinene/isoprene) | - | - | - | - | 30 | - | - | - | - | - | - | - |
| | Synthesis Example L (pyrogallol/styrene) | - | - | - | - | - | - | - | - | - | - | - | - |
| | Synthesis Example M (pyrocatechol/isoprene) | - | - | - | - | - | 30 | - | - | - | - | - | - |
| | Synthesis Example N (vinylbenzoic acid/β-pinene) | - | - | - | - | - | - | 30 | - | - | - | - | - |
| | Synthesis Example O (trimethoxybenzene/β-pinene) | - | - | - | - | - | - | - | 30 | - | - | - | - |
| Tackifier resin (T2) [% by weight] | PX1150 (terpene resin) | - | 40 | 90 | 100 | - | - | - | - | - | - | - | - |
| | KE359 (rosin ester resin) | - | - | - | - | - | - | - | - | - | - | 30 | - |
| | G150 (terpene phenolic resin) | - | - | - | - | - | - | - | - | - | - | - | 30 |
| Physical properties | Shear storage modulus (25°C) [Pa/$10^5$] | 1.9 | 2.3 | 3.5 | 6.0 | 1.8 | 1.3 | 1.7 | 1.7 | 2.1 | 1.9 | 2.0 | 2.2 |
| | Loss tangent peak temperature [°C] | -29 | -10 | 18 | 22 | -33 | -38 | -31 | -31 | -29 | -30 | -25 | -16 |
| Evaluation | 180° Peel test for SUS | ○ | ○○ | ○○ | ○ | ○ | ○ | ○○ | ○○ | ○ | ○ | ○ | ○ |
| | 180° Peel test for PP | ○ | ○○ | ○○ | ○ | ○ | Δ | ○○ | ○○ | Δ | ○ | × | × |
| | 180° Peel test for PTFE | Δ | ○○ | ○○ | Δ | Δ | Δ | ○○ | ○ | Δ | Δ | × | × |
| | Alkali resistance test | ○ | ○ | ○ | ○ | ○ | Δ | ○ | ○ | ○ | ○ | × | Δ |

INDUSTRIAL APPLICABILITY

The present invention can provide a compound capable of increasing the adhesion strength of adhesive compositions, particularly, even to low polarity adherends. The present invention can also provide a method for producing the compound, an adhesive composition containing the compound, and an adhesive tape including an adhesive layer containing the adhesive composition.

## Claims

1. A compound comprising:

   a structural unit (A) derived from a monomer (a) having a solvation free energy $\Delta\mu$ with polytetrafluoroethylene of -30 kcal/mol or less; and
   a structural unit (B) derived from at least one monomer (b) selected from the group consisting of a terpene monomer, a vinyl monomer, and a conjugated diene monomer.

2. The compound according to claim 1, having a Young's modulus at 25°C of 10 MPa or greater.

3. The compound according to claim 1 or 2, further comprising an aliphatic hydrocarbon group containing an unsaturated double bond.

4. The compound according to claim 1, 2, or 3, having a structural unit (A) content of 1 mol% or greater and 60 mol% or less.

5. The compound according to claim 1, 2, 3, or 4, having a weight average molecular weight of 400 or greater and 10,000 or less.

6. The compound according to claim 1, 2, 3, 4, or 5, having a glass transition temperature of 0°C or higher and 200°C or lower.

7. The compound according to claim 1, 2, 3, 4, 5, or 6,
   wherein a bio-derived carbon content in carbon in the compound is 10% or greater.

8. The compound according to claim 1, 2, 3, 4, 5, 6, or 7,
   wherein the structural unit (A) is at least one selected from the group consisting of a structural unit (A-1) and a structural unit (A-1') that are represented by the following formulas:

   [Chem. 1]

   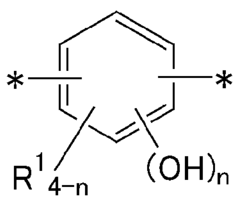 (A-1) 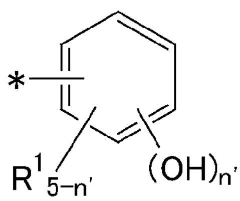 (A-1')

   wherein each $R^1$ represents a hydrogen atom, an aliphatic hydrocarbon group, an aromatic hydrocarbon group, a polar functional group, an aliphatic hydrocarbon group containing a polar functional group, or an aromatic hydrocarbon group containing a polar functional group; n represents an integer of 2 or greater and 4 or less; and n' represents an integer of 2 or greater and 5 or less.

9. The compound according to claim 8,
   wherein in the structural unit (A-1) and the structural unit (A-1'), n and n' are 2.

10. The compound according to claim 8,
    wherein in the structural unit (A-1) and the structural unit (A-1'), n and n' are 3.

**11.** The compound according to claim 1, 2, 3, 4, 5, 6, or 7,
wherein the structural unit (A) is at least one selected from the group consisting of a structural unit (A-2), a structural unit (A-2'), a structural unit (A-3), a structural unit (A-3'), a structural unit (A-4), and a structural unit (A-4') that are represented by the following formulas:

[Chem. 2]

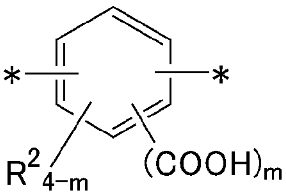

(A-2)

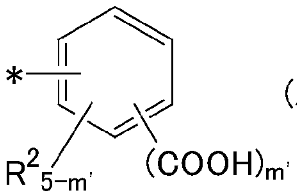

(A-2')

[Chem. 3]

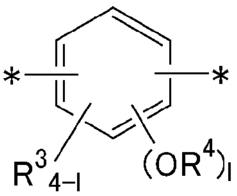

(A-3)

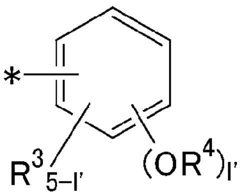

(A-3')

[Chem. 4]

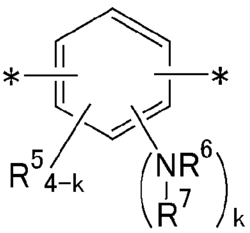

(A-4)

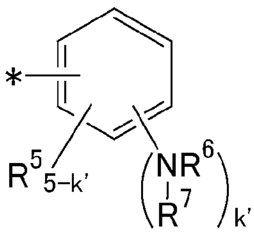

(A-4')

wherein each $R^2$, each $R^3$, and each $R^5$ represents a hydrogen atom, an aliphatic hydrocarbon group, an aromatic hydrocarbon group, a polar functional group, an aliphatic hydrocarbon group containing a polar functional group, or an aromatic hydrocarbon group containing a polar functional group; each $R^4$ represents an aliphatic hydrocarbon group, an aromatic hydrocarbon group, an aliphatic hydrocarbon group containing a polar functional group, or an aromatic hydrocarbon group containing a polar functional group; each $R^6$ and each $R^7$ represents a hydrogen atom or an aliphatic hydrocarbon group; m represents an integer of 1 or greater and 4 or less; m' represents an integer of 1 or greater and 5 or less; l represents an integer of 2 or greater and 4 or less; l' represents an integer of 2 or greater and 5 or
less; k represents an integer of 1 or greater and 4 or less; and k' represents an integer of 1 or greater and 5 or less.

**12.** The compound according to claim 1, 2, 3, 4, 5, 6, 7, 8, 9, 10, or 11,
wherein the structural unit (A) is in a backbone structure or at a terminal of the backbone structure.

**13.** A method for producing a compound including a structural unit (A) derived from a monomer (a) having a solvation free energy $\Delta\mu$ with polytetrafluoroethylene of - 30 kcal/mol or less and a structural unit (B) derived from at least one monomer (b) selected from the group consisting of a terpene monomer, a vinyl monomer, and a conjugated diene monomer, the method comprising
copolymerizing the monomer (a) and the monomer (b).

**14.** The method for producing a compound according to claim 13,
wherein the monomer (a) is at least one selected from the group consisting of a monomer (a-1), a monomer (a-2), a monomer (a-3), and a monomer (a-4) that are represented by the following formulas:

[Chem. 5]

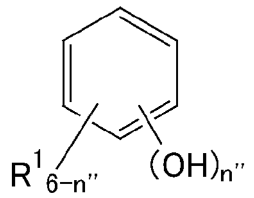

(a-1)

wherein each $R^1$ represents a hydrogen atom, an aliphatic hydrocarbon group, an aromatic hydrocarbon group, a polar functional group, an aliphatic hydrocarbon group containing a polar functional group, or an aromatic hydrocarbon group containing a polar functional group; and n" represents an integer of 2 or greater and 5 or less, and

[Chem. 6]

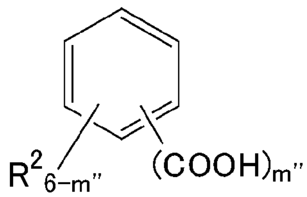

(a-2)

[Chem. 7]

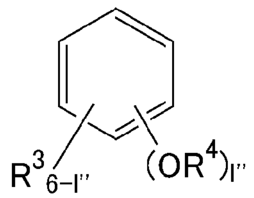

(a-3)

[Chem. 8]

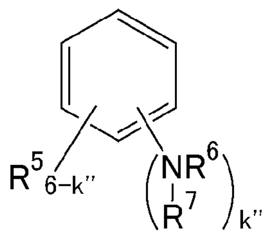

(a-4)

wherein each $R^2$, each $R^3$, and each $R^5$ represents a hydrogen atom, an aliphatic hydrocarbon group, an aromatic hydrocarbon group, a polar functional group, an aliphatic hydrocarbon group containing a polar functional group, or an aromatic hydrocarbon group containing a polar functional group; each $R^4$ represents an aliphatic hydrocarbon group, an aromatic hydrocarbon group, an aliphatic hydrocarbon group containing a polar functional group, or an aromatic hydrocarbon group containing a polar functional group; each $R^6$ and each $R^7$ represents a hydrogen atom or an aliphatic hydrocarbon group; m" represents an integer of 1 or greater and 5 or less; l" represents an integer of 2 or greater and 5 or less; and k" represents an integer of 1 or greater and 5 or less.

**15.** The method for producing a compound according to claim 13 or 14, wherein the monomer (a) and the monomer (b) are copolymerized by cationic polymerization.

**16.** An adhesive composition comprising:

a base polymer; and
the compound (T1) according to claim 1, 2, 3, 4, 5, 6, 7, 8, 9, 10, 11, or 12.

**17.** The adhesive composition according to claim 16, wherein the compound (T1) according to claim 1, 2, 3, 4, 5, 6, 7, 8, 9, 10, 11, or 12 is contained in an amount of 1 part by weight or greater and 35 parts by weight or less relative to 100 parts by weight of the base polymer.

**18.** The adhesive composition according to claim 16 or 17, further comprising at least one tackifier resin (T2) selected from the group consisting of a rosin ester resin, a terpene resin, and a petroleum resin.

**19.** The adhesive composition according to claim 18, wherein the tackifier resin (T2) is contained in an amount of 10 parts by weight or greater and 100 parts by weight or less relative to 100 parts by weight of the base polymer.

**20.** The adhesive composition according to claim 16, 17, 18, or 19, wherein the base polymer is an acrylic polymer.

**21.** The adhesive composition according to claim 20, wherein the acrylic polymer includes a structural unit derived from a monomer containing a crosslinkable functional group.

**22.** The adhesive composition according to claim 21, wherein the structural unit derived from a monomer containing a crosslinkable functional group is contained in an amount of 0.01% by weight or greater and 20% by weight or less in the acrylic polymer.

**23.** The adhesive composition according to claim 20, 21, or 22, wherein the acrylic polymer has a weight average molecular weight of 200,000 or greater and 2,000,000 or less.

**24.** The adhesive composition according to claim 16, 17, 18, or 19, wherein the base polymer is a styrene elastomer that is a block copolymer including a block derived from a styrene monomer and a block derived from a conjugated diene monomer or a hydrogenated product of the block copolymer.

**25.** The adhesive composition according to claim 24, wherein the styrene elastomer is a styrene-isoprene-styrene (SIS) block copolymer or a styrene-butadienestyrene (SBS) block copolymer.

**26.** The adhesive composition according to claim 24 or 25, wherein the styrene elastomer has a diblock proportion of 50% by weight or greater.

**27.** The adhesive composition according to claim 24, 25, or 26, wherein the styrene elastomer has a styrene content of 20% by weight or less.

**28.** An adhesive tape comprising an adhesive layer containing the adhesive composition according to claim 16, 17, 18, 19, 20, 21, 22, 23, 24, 25, 26, or 27.

**29.** The adhesive tape according to claim 28, comprising an adhesive layer containing the adhesive composition according to claim 20, 21, 22, or 23, wherein the adhesive layer has a shear storage modulus at 25°C of $1.0 \times 10^4$ Pa or greater and $5.0 \times 10^5$ Pa or less as measured using a dynamic viscoelastometer at a measurement frequency of 10 Hz.

**30.** The adhesive tape according to claim 29, wherein a loss tangent of the adhesive layer has a peak at a temperature of -20°C or higher and 20°C or lower as measured using a dynamic viscoelastometer at a measurement frequency of 10 Hz.

**31.** The adhesive tape according to claim 28, 29, or 30, used for fixing an electronic device component or an in-vehicle component.

## INTERNATIONAL SEARCH REPORT

International application No.
**PCT/JP2021/031585**

### A. CLASSIFICATION OF SUBJECT MATTER

***C08G 61/02***(2006.01)i; ***C08G 61/00***(2006.01)i; ***C08L 33/04***(2006.01)i; ***C08L 45/00***(2006.01)i; ***C08L 53/00***(2006.01)i; ***C08L 53/02***(2006.01)i; ***C08L 57/02***(2006.01)i; ***C08L 65/00***(2006.01)i; ***C08L 93/04***(2006.01)i; ***C08L 101/00***(2006.01)i; ***C09J 7/38***(2018.01)i; ***C09J 11/08***(2006.01)i; ***C09J 133/00***(2006.01)i; ***C09J 153/02***(2006.01)i; ***C09J 201/00***(2006.01)i
FI: C08G61/02; C09J133/00; C09J7/38; C08L101/00; C08L65/00; C08L45/00; C08L57/02; C08L93/04; C08L33/04; C09J153/02; C08L53/00; C08L53/02; C09J11/08; C09J201/00; C08G61/00

According to International Patent Classification (IPC) or to both national classification and IPC

### B. FIELDS SEARCHED

Minimum documentation searched (classification system followed by classification symbols)

C08G2/00-2/38; C08G61/00-61/12; C08K3/00-13/08; C08L1/00-101/14; C09J7/00-7/50; C09J1/00-5/10; C09J9/00-201/10

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | JP 10-212391 A (IDEMITSU PETROCHEM CO LTD) 11 August 1998 (1998-08-11) claims, paragraphs [0009], [0023]-[0031], examples | 1-10, 12-16, 18-19, 24-31 |
| A | | 11, 17, 20-23 |
| X | JP 11-130820 A (IDEMITSU PETROCHEM CO LTD) 18 May 1999 (1999-05-18) claims, paragraphs [0009], [0056]-[0062], examples | 1-10, 12-31 |
| A | | 11 |
| X | JP 2017-160294 A (SANYO CHEMICAL IND LTD) 14 September 2017 (2017-09-14) claims, example 8 | 1-7, 11-15 |
| A | | 8-10, 16-31 |
| A | JP 2003-301153 A (ASAHI KASEI CORP) 21 October 2003 (2003-10-21) entire text | 1-31 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 October 2021** | **26 October 2021** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No.
**PCT/JP2021/031585**

**C. DOCUMENTS CONSIDERED TO BE RELEVANT**

| Category* | Citation of document, with indication, where appropriate, of the relevant passages | Relevant to claim No. |
|---|---|---|
| A | JP 2007-106954 A (KRATON JSR ELASTOMERS KK) 26 April 2007 (2007-04-26) entire text | 1-31 |
| A | JP 2015-227395 A (SOKEN KAGAKU KK) 17 December 2015 (2015-12-17) entire text | 1-31 |

Form PCT/ISA/210 (second sheet) (January 2015)

**INTERNATIONAL SEARCH REPORT**
**Information on patent family members**

International application No.
**PCT/JP2021/031585**

| Patent document cited in search report | Publication date (day/month/year) | Patent family member(s) | Publication date (day/month/year) |
|---|---|---|---|
| JP 10-212391 A | 11 August 1998 | (Family: none) | |
| JP 11-130820 A | 18 May 1999 | US 6040388 A<br>claims, column 2, lines 9-18, column 8, line 53 to column 9, line 35, examples<br>EP 845484 A2<br>TW 464657 B<br>KR 10-0541421 B1<br>CN 1183420 A | |
| JP 2017-160294 A | 14 September 2017 | (Family: none) | |
| JP 2003-301153 A | 21 October 2003 | (Family: none) | |
| JP 2007-106954 A | 26 April 2007 | (Family: none) | |
| JP 2015-227395 A | 17 December 2015 | TW 201604256 A<br>CN 106414643 A<br>KR 10-2017-0013858 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015052050 A
- JP 2015021067 A
- JP 2015120876 A


### Non-patent literature cited in the description


- A crystal structure of ultra-dispersed form of polytetrafluoroethylene based on X-ray powder diffraction data. *Powder Diffr.,* September 2004, vol. 19 (3